# EUROPÄISCHE PATENTSCHRIFT

(11) **EP 1 862 931 B1**
(45) Veröffentlichungstag und Bekanntmachung des Hinweises auf die Patenterteilung: **03.11.2010**
(21) Anmeldenummer: 07109289.4
(22) Anmeldetag: 31.05.2007
(51) Int. Cl.: G06F 19/00, H04L 29/06

(54) **Vorrichtung und Verfahren zum Schutz eines medizinischen Geräts und eines von diesem Gerät behandelten Patienten vor gefährdenden Einflüssen aus einem Kommunikationsnetzwerk**
Device and method for protecting a medical device and a patient being treated with one of these devices against dangerous effects from a communications network
Dispositif et procédé destinés à la protection d'un appareil médical et d'un patient traité par cet appareil contre les actions dangereuses d'un réseau de communication

(30) Priorität: 03.06.2006 DE 102006026088; 25.05.2007 DE 102007024720
(43) Veröffentlichungstag der Anmeldung: 05.12.2007
(60) Teilanmeldung: 10172615.6
(73) Patentinhaber: B. Braun Medizinelektronik GmbH & Co. KG, 82178 Puchheim (DE)
(72) Erfinder: Steinkogler, Alexander, Dr., 81249 München (DE); Lauer, Hans-Martin, 80997 München (DE); Preus, Niko, 95502 Himmelkron (DE)
(74) Vertreter: Hannke, Christian

(56) Entgegenhaltungen:
- WO-A-03/095024
- US-A- 6 026 502

## Beschreibung

Die vorliegende Erfindung betrifft eine Vorrichtung zur Integration in ein medizinisches Gerät, welches zur Einbindung in ein Kommunikationsnetzwerk, das zumindest einen unsicheren Bereich sowie geräteseitig einen sicheren Bereich aufweist, geeignet ist. Weiterhin betrifft die vorliegende Erfindung ein medizinisches Gerät an sich, welches zur Einbindung in ein Kommunikationsnetzwerk mit zumindest einem unsicheren Bereich und geräteseitig einem sicheren Bereich geeignet ist, sowie ein medizinisches System mit einer Vielzahl von derartigen medizinischen Geräten oder Teilgeräten. Zudem betrifft die Erfindung ein Verfahren zur Steuerung einer entsprechenden Vorrichtung zur Integration in ein medizinisches Gerät.

Im medizinischen Bereich wurden in der Vergangenheit die meisten Geräte als Stand-Alone-Geräte konzipiert. Heute wird jedoch ein wachsender Anteil dieser Geräte in Kommunikationsnetzwerke eingebunden. Die Ursache hierfür liegt teilweise in einem wachsenden Kostenbewusstsein in medizinischen Einrichtungen, die eine Verbesserung der verwendeten Prozesse, teilweise zentrale Datenspeicherung und damit den Einsatz von Kommunikationsnetzwerken nötig machen, aber auch in der wachsenden Komplexität von medizinischen Geräten, die eine Realisierung mit einem einzelnen Computersystem nicht oder sehr aufwendig realisierbar machen. Daher sind oft einzelne komplexe medizinische Geräte de facto Systeme, die auf einer Anzahl einzelner Computer-Subsysteme basieren, welche über ein Kommunikationsnetzwerk miteinander verbunden sind und gemeinsam zur Gesamtfunktionalität des medizinischen Geräts beitragen. Es gibt andererseits viele aus einzelnen medizinischen Geräten zusammengesetzte Systeme, die erst durch das Zusammenwirken der einzelnen im System enthaltenen medizinischer Geräte eine Gesamtfunktionalität erbringen, die von den einzelnen Geräten nicht erbracht werden kann. Somit stellen diese Systeme medizinischer Geräte selbst wiederum medizinische Geräte dar, die eine höhere Komplexibilität als die enthaltenen medizinischen Subsysteme/-geräte aufweisen. Zum Beispiel kann im Bereich von Infusionspumpen ein deutlicher Mehrwert entstehen, wenn Pumpen zu einem Pumpensystem zusammen gefügt werden können, innerhalb dessen die Pumpen miteinander kommunizieren. Wenn ein solches Pumpensystem auch noch mit der Außenwelt kommunizieren kann, ermöglicht dies, die Infusionsdaten, welche von dem vom Pumpensystem mit Infusionen versorgten Patienten anfallen, an ein Krankenhausinformationssystem weiterzuleiten. Es ist dann möglich, das Infusionspumpensystem als ein medizinisches Gerät im Sinne der hier beschriebenen Erfindung anzusehen.

Auch findet in den letzten Jahren aus Gründen der Kostenreduktion eine zunehmende Verlagerung von Patienten aus dem stationären in den ambulanten Bereich statt, die jedoch nur dann zu einer durchschlagenden Verringerung der entstehenden Kosten führen kann, wenn die entstehenden medizinischen Daten zu Zwecken der Analyse und Auswertung an die entsprechende medizinische Einrichtung zeitnah und effizient übertragen werden können. Auch dies geschieht im zunehmenden Maße mit Hilfe von Kommunikationsnetzwerken.

In diesem Kontext treten jedoch eine Reihe von Schwierigkeiten und Gefahrenquellen auf, die insbesondere durch die bei der Behandlung von Patienten bzw. bei der Bedienung von medizinischen Geräten notwendige hohe Sicherheit von gesteigerter Wichtigkeit sind. Durch den Einsatz von Kommunikationsnetzwerken in diesem Bereich entsteht daher auch eine neue Art von Gefährdungen für den Patienten respektive den Bediener von medizinischen Geräten. Es ist möglich, dass durch eine Einwirkung, die vom Kommunikationsnetz ausgeht, das medizinische Gerät derart gestört wird, dass dies zu einer Gefährdung des behandelten Patienten oder zu einer Fehlfunktion des medizinischen Geräts führen kann. Eine solche Einwirkung, die zu einer möglichen Gefährdung durch das medizinische Gerät führen kann, wird im Folgenden als Attacke auf das medizinische Gerät bezeichnet. Eine solche Attacke kann z. B. durch Software verursacht werden, die bewusst versucht, Sicherheitslücken auszuschöpfen, um an vermeintlich vorhandene, kriminell nutzbare vertrauliche Daten des im medizinischen Gerät enthaltenen Computersystems zu gelangen oder solche zu verfälschen. Derartige Software wird im Folgenden als Malware bezeichnet. Da Malware die Zielteile der Funktionalität eines Computersystems korrumpiert, um die Daten auszuspionieren oder zu verfälschen, muss hier in besonders hohem Maße von einem Risiko für den Patienten bzw. die korrekte Funktion des medizinischen Geräts ausgegangen werden, wenn dieses von Malware attackiert wird.

Um die Datensicherheit und Geheimhaltung der im Netzwerk enthaltenen Daten gewährleisten zu können, wird üblicherweise beim Entwurf von Kommunikationsnetzwerken von Teilen des Kommunikationsnetzwerkes mit unterschiedlichen Sicherheitsniveau ausgegangen. An den Trennstellen zwischen diesen Bereichen werden üblicherweise Sicherungsmechanismen, wie z. B. Firewalls, installiert. Eine solche Firewall wird beispielsweise in WO 03/095024 und US 6026502 offenbart. Der Schwerpunkt einer derartigen Absicherung der oben genannten Sicherungsmechanismen liegt jedoch auf der kommerziell in hohem Maße interessanten Absicherung von Datensicherheit und -geheimhaltung und ist nicht oder nur unvollkommen an die von medizinischen Geräten herrührenden Anforderungen angepasst.

Es ist sogar möglich, dass eine Attacke auf ein medizinisches Gerät durch andere Kommunikationspartner, also in der Regel medizinische Geräte, innerhalb des als sicher angesehenen Teils eines Kommunikationsnetzes entsteht, obwohl die einzelnen Kommunikationspartner fehlerfrei arbeiten und ein von Ihnen als kooperativ angesehenes Kommunikationsverhalten aufweisen, das sich jedoch in der Summe und in bestimmten Situationen zu einer Störung ausweiten kann. Dies ist vergleichbar zu Staus auf einer Autobahn, die ab einer gewissen Fahrzeugdichte spontan, also ohne erkennbare äußere Ursache, entstehen. Alternativ kann eine Attacke auf ein medizinisches Gerät durch andere medizinische Geräte innerhalb des als sicher angesehenen Teils eines Kommunikationsnetzes entstehen, wenn die Kommunikationsprotolle zweier Klassen medizinischer Geräte zu möglichen Fehlinterpretationen führen oder durch die Kommunikation der medizinischen Geräte das andere medizinische Gerät überlastet wird.

In besonderem Maße erhöht sich potentielle Gefährdung dann, wenn zur Erstellung des medizinischen Geräts fertige Allround-Software-Komponenten, wie z. B. geläufige Betriebssysteme verwendet werden, die für eine Anzahl möglicher Anwendungsfälle entwickelt wurden, eine hohe inherente Komplexität aufweisen, und daher im besonderen Maße dem Risiko einer Attacke unterworfen sind. Der Hersteller eines medizinischen Geräts befindet sich hier in dem Dilemma, dass einerseits die verwendete Software-Komponente eine potentielle Gefährdung darstellt, dass aber andererseits eine Entwicklung der medizinischen Geräte ohne Verwendung solcher Komponenten so komplex ist, dass dadurch ein hohes potentielles Risiko für den Patienten oder die korrekte Funktion des medizinischen Geräts ausgeht. Was die Verwendung solcher Software-Komponenten noch riskanter macht, ist die Tatsache, dass Malware meist gezielt für solche Software-Komponenten geschrieben wird und daher dort vorhandene Sicherheitslücken gezielt ausnutzt. Dabei kann die Malware entweder auf indirektem Wege über eine Verbindung des sicheren Teils des Kommunikationsnetzes zu einem unsicheren Teil oder aber auf direktem Weg über Datenträger oder eine Kombination beider Wege in das medizinische Gerät gelangen.

Es ist das Ziel der vorliegenden Erfindung, diese Schwierigkeiten zu beheben respektive zumindest teilweise zu lösen und eine Vorrichtung bereitzustellen, welche medizinische Geräte vor den vorgenannten gefährdenden Einflüssen aus einem Kommunikationsnetzwerk zu schützen erlaubt.

Die Erfindung wird durch die Ansprüche definiert.

Die vorliegende Erfindung erlaubt es, auf die speziellen Anforderungen einzugehen, die von medizinischen Geräten an eine Absicherung gegenüber Attacken aus dem Netzwerk oder sonstigen Fehlfunktionen gestellt werden, und betrifft eine Vorrichtung laut dem Oberbegriff des Anspruchs 1, die Übertragungsmittel zur Übertragung von Kommunikationspaketen zu und von dem medizinischen Gerät über das Kommunikationsnetzwerk aufweist, die Überwachungsmittel aufweist, um den Zustand der Verbindung des Geräts mit dem Netzwerk zu überwachen, und die Unterbrechungsmittel aufweist, um eine bestehende Verbindung zwischen dem sicheren und unsicheren Bereich des Netzwerks zu unterbrechen, falls während der Überwachung ein Zustand der Netzwerkverbindung detektiert wird, der eine Gefährdung für einen vom Gerät behandelten Patienten repektive für die korrekte Funktion des Geräts darstellt. Dies hat den Vorteil, dass aufgrund der besonderen Anforderungen im medizinischen Bereich sowie über die üblichen Eigenschaften typischer Firewalls hinausgehend insbesondere die Verbindung des sicheren Bereichs des Kommunikationsnetzwerks zum unsicheren Bereich komplett getrennt werden kann, falls eine Gefährdung irgendwelcher Art für den Patienten oder das Gerät festgestellt wird.

In einer bevorzugten Ausprägung der erfindungsgemäßen Vorrichtung verfügen die Übertragungsmittel über einen Paketfilter, der eine Paketfilterung bei den zwischen dem unsicheren Bereich und dem sicheren Bereich des Kommunikationsnetzwerks übertragenen Kommunikationspaketen ausführt, wobei der Paketfilter geeignet ist, für das medizinische Gerät potentiell gefährdende Kommunikationspakte nicht passieren zu lassen. Weiterhin können die Unterbrechungsmittel durch zumindest einen Unterbrechungsschalter realisiert sein sowie die Überwachungsmittel zumindest eine Steuerungslogik aufweisen, die bei Feststellung eines Zustands der Netzwerkverbindung, der eine Gefährdung für einen Patienten oder für die korrekte Funktion des Gerätes darstellt, die Unterbrechung des oder der Unterbrechungsschalter auslöst, um den unsicheren Bereich vom direkt an das medizinische Gerät angeschlossenen sicheren Bereich des Kommunikationsnetzwerks abzutrennen.

Der vorgenannte Paketfilter kann derart gestaltet werden, dass er eine bidirektionale Paketfilterung bei den zwischen dem unsicheren Bereich und dem sicheren Bereich des Kommunikationsnetzwerks übertragenen Kommunikationspaketen auszuführen erlaubt. Dies gewährleistet somit eine Kontrolle und Filterung der übertragenen Daten in beiden Richtungen. Zudem kann in diesem Falle die Trennung zwischen sicherem Bereich und unsicherem Bereich des Kommunikationsnetzwerks bevorzugt als logische Segmentierung ausgeführt sein.

Dadurch kann der Paketfilter insbesondere teilweise oder vollständig die vorgenannten Überwachungsmittel und/oder die Unterbrechungsmittel ersetzen, in dem er die Fährten der Kommunikationspakete oder alle Kommunikationspakete beim Filtervorgang nicht passieren lässt.

Die Trennung zwischen sicherem Bereich und unsicherem Bereich des Kommunikationsnetzwerks kann jedoch auch als physikalische Segmentierung ausgeführt sein, insbesondere in Form unterschiedlicher physikalischer Übertragungswege im sicheren bzw. im unsicheren Bereich des Netzwerks oder durch unterschiedliche Instanzen desselben Übertragungswegs in diesem Bereich. Falls die im Kommunikationsnetzwerk Verwendung findenden Übertragungswege kabelgebunden sind, ist es von besonderem Vorteil, Lichtwellenleiter für die Übertragungswege zu benutzten, so wie dies ausführlich in der nachfolgenden Beschreibung dargestellt und begründet werden wird.

In einer weiteren Ausführungsform der vorliegenden Vorrichtung besitzt die besagte Steuerungslogik Mittel zur Durchführung einer statischen - und/oder einer dynamischen Prüfung der im Kommunikationsnetzwerk zu übertragenden Daten, deren Ergebnis zur Aufrechterhaltung oder Trennung einer bestehenden Verbindung zwischen dem unsicheren Bereich und dem sicheren Bereich des Netzwerks durch Auslösen der/des Unterbrechungsschalters führt.

Der vorgenannte Unterbrechungsschalter kann auf der an den sicheren Bereich des Kommunikationsnetzwerks angeschlossenen Seite und/oder auf der an den unsicheren Bereich des Kommunikationsnetzwerks angeschlossenen Seite der erfindungsgemäßen Vorrichtung angebracht sein. Dadurch ist es möglich, nur das medizinische Gerät und/oder sowohl das medizinische Gerät als auch die Vorrichtung selbst vom unsicheren Bereich des Netzes abzutrennen.

In einer besonders vorteilhaften Ausführungsform einer erfindungsgemäßen Vorrichtung besitzt diese eine redundante Architektur, indem in den Überwachungsmitteln ein Modell der Funktionen der Übertragungsmittel integriert ist, das deren korrekte Funktionsweise zu überprüfen erlaubt. Eine erfindungsgemäße Vorrichtung kann auch mindestens zwei diversitäre Kanäle mit jeweils eigenen Übertragungsmitteln, Überwachungsmitteln sowie Unterbrechungsmitteln aufweisen, wobei jeder Kanal sowohl sich als auch den anderen Kanal unabhängig überwachen und bei Feststellung eines Zustands der Netzwerkverbindung, der eine Gefährdung für den Patienten respektive für die komplette Funktion des Geräts darstellt, vom unsicheren Bereich des Kommunikationsnetzwerks trennen kann. Die beiden vorgenannten Ausführungsformen einer erfindungsgemäßen Vorrichtung zeichnen sich durch eine besonders hohe Sicherheit gegenüber Attacken aus dem unsicheren Bereich des Kommunikationsnetzwerks oder sonstigen Fehlfunktionen aus.

Die vorliegende Erfindung ist weiterhin auf ein medizinisches Gerät an sich gerichtet, das zur Einbindung an ein Kommunikationsnetzwerk, welches zumindest einen unsicheren Bereich sowie geräteseitig einen sicheren Bereich aufweist, geeignet ist, und das eine erfindungsgemäße Vorrichtung gemäß einer der vorstehend erwähnten Ausprägungen aufweist. Bei dem medizinischen Gerät kann es sich insbesondere um Infusionspumpen oder Patientenmonitore handeln.

Die vorliegende Erfindung richtet sich auch auf medizinische Systeme mit einer Vielzahl von medizinischen Geräten der vorgenannten Art oder solchen Teilgeräten, wobei das System zumindest eine wie vorstehend erwähnte erfindungsgemäße Vorrichtung aufweist.

Schließlich richtet sich die vorliegende Erfindung auf ein Verfahren zur Steuerung einer derartigen Vorrichtung, wobei das Verfahren die Übertragung von Kommunikationspakten zu und von dem medizinischen Gerät über das Kommunikationsnetz sicherstellt, es den Zustand der Verbindung des Gerätes zum Netzwerk überwacht, und es eine bestehende Verbindung zwischen dem sicheren Bereich und dem unsicheren Bereich des Netzwerks unterbricht, falls während der Überwachung ein Zustand der Netzwerkverbindung festgestellt wird, der eine Gefährdung für den Patienten respektive für die korrekte Funktion des Geräts darstellt. Die verschiedenen Ausprägungen und Vorteile eines derartigen Verfahrens werden in der nachfolgenden Beschreibung im Detail erläutert werden.

Die beiliegenden Figuren stellen beispielhaft mehrere Ausführungsformen einer erfindungsgemäßen Vorrichtung, eines erfindungsgemäßen medizinischen Geräts bzw. Systems, und eines erfindungsgemäßen Verfahrens dar.
- Fig. 1: stellt die im Stand der Technik übliche Anbindung eines in der Wohnung eines Patienten befindlichen medizinischen Geräts in der Wohnung eines Patienten dar;
- Fig. 2: illustriert schematisch und beispielhaft das Prinzip einer erfindungsgemäßen Vorrichtung;
- Fig. 3: stellt eine erfindungsgemäße Vorrichtung mit bidirektionaler Paketfilterung in beispielhafter Art dar;
- Fig.4: gibt skizzenhaft den Algorithmus eines in einer erfindungsgemäßen Vorrichtung verwendeten Paketfilters wieder;
- Fig. 5: stellt die Abtrennung von Patientenmonitoren von einer Zentrale dar;
- Fig. 6: stellt beispielhaft den Aufbau eines Systems medizinischer Geräte dar, die durch einen unsicheren Teil eines Kommunikationsnetzwerks miteinander verbunden sind;
- Fig. 7: illustriert die Verwendung modifizierter Kommunikationspakte im ungesicherten Teil des Kommunikationsnetzes;
- Fig. 8: zeigt die Verwendung verschlüsselter Kommunikationspakete im ungesicherten Teil des Kommunikationsnetzwerks;
- Fig. 9: stellt eine Konfiguration, in der die erfindungsgemäße Vorrichtung vom unsicheren Teil des Kommunikationsnetzes getrennt werden kann, dar;
- Fig. 10: illustriert eine Konfiguration, welche die beidseitige Trennung der erfindungsgemäßen Vorrichtung ermöglicht;
- Fig. 11: zeigt ein Beispiel für eine redundante Architektur einer erfindungsgemäßen Vorrichtung;
- Fig. 12: zeigt eine Konfiguration einer erfindungsgemäßen Vorrichtung mit einer diversitär redundanten Architektur;
- Fig. 13: illustriert eine mögliche diversitäre Realisierung der erfindungsgemäßen Vorrichtung;
- Fig. 14: zeigt in beispielhafter Weise die Absicherung eines Systems von Infusionspumpen gegen Einflüsse aus dem unsicheren Bereich des Kommunikationsnetzwerks;
- Fig. 15: zeigt die herkömmliche Art und Weise der Anbindung von Patientenmonitoren an einen zentralen Monitor;
- Fig. 16: illustriert die Anbindung von Patientenmonitoren an einen Zentralmonitor über erfindungsgemäße Vorrichtungen;
- Fig. 17: illustriert schematisch und beispielhaft eine modular aufgebaute erfindungsgemäße Vorrichtung;
- Fig. 18: illustriert beispielhaft eine hierarchische Absicherung einer medizinischen Einrichtung mittels der vorliegenden Erfindung;

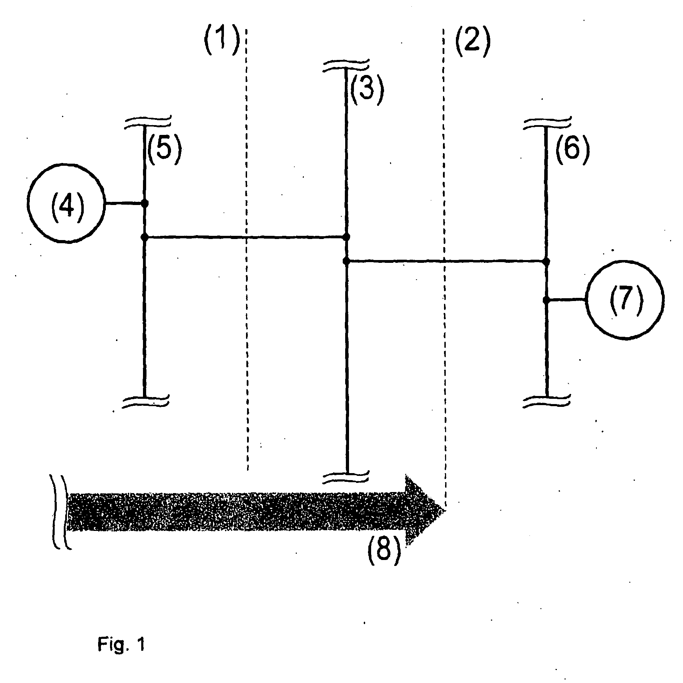

Im Nachfolgenden wird nunmehr die vorliegende Erfindung unter Verweis auf die vorstehenden Figuren im Detail erläutert werden. Zunächst sei dabei auf Fig. 1 verwiesen, welche die bisher übliche Anbindung eines datenerfassenden medizinischen Geräts, das sich in der Wohnung des Patienten befindet, darstellt. Dabei sind die Wohnung 1 des Patienten und die medizinische Einrichtung 2, deren Systemgrenzen durch gestrichelte Linien angedeutet sind, über ein WAN-Netzwerk 3, z. B. das Internet, miteinander verbunden. Das datenerfassende medizinische Gerät 4 ist über das LAN-Netzwerk 5 an das WAN-Netzwerk 3 angekoppelt. Daran ist das LAN-Netzwerk 6 der medizinischen Einrichtung und an jenes das auswertende medizinische Gerät 7 angekoppelt. Der aus Sicht der medizinischen Einrichtung unsichere Bereich 8 des Kommunikationsnetzwerks ist in Fig. 1 durch einen grauen Pfeil symbolisiert. Das Ziel der vorliegenden Erfindung ist es, dass im sicheren Bereich des Kommunikationsnetzwerks angebrachte medizinische Gerät gegen Einflüsse aus dem unsicheren Bereich 8 des Netzwerks zu schützen. Dies soll auf einfache und effiziente Art und Weise realisiert werden und dennoch die im medizinischen Bereich üblichen hohen Sicherheitsstandards erfüllen.

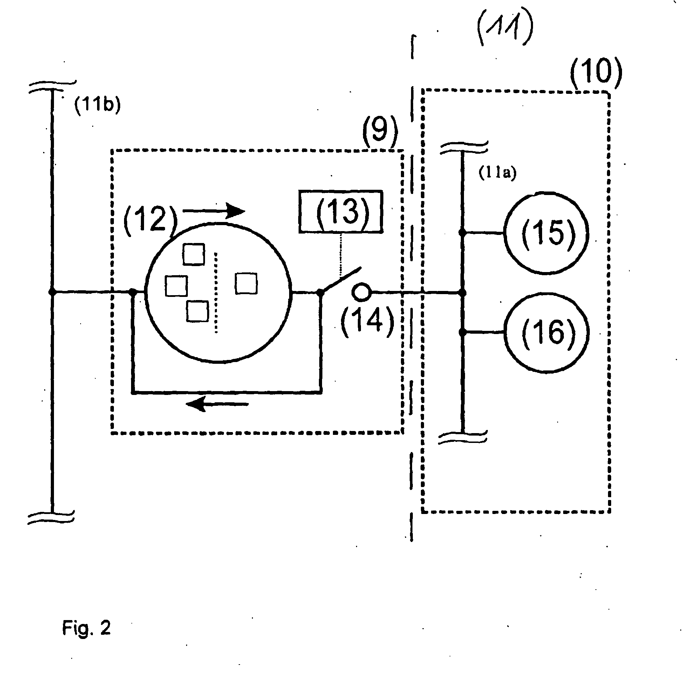

In Fig. 2 ist eine schematische Darstellung der erfindungsgemäßen Vorrichtung wiedergegeben. Die erfindungsgemäße Vorrichtung 9 sichert ein medizinisches Gerät 10 gegen mögliche Attacken, die aus dem unsicheren Bereich 11 b des Kommunikationsnetzes 11 kommen können, ab. Es bedient sich dazu auf dem Weg vom unsicheren Teil des Kommunikationsnetzes 11 zum medizinischen Gerät eines Paketfilters 12, der Kommunikationspakete, welche das medizinische Gerät 10 potentiell gefährden könnten, nicht passieren lässt. Eine Steuerungslogik 13 ermöglicht es, den unsicheren Bereich 11 b des Kommunikationsnetzes 11 vom medizinischen Gerät 10 mit Hilfe des Unterbrechungsschalters 14 abzutrennen. Dabei ist hier beispielhaft eine Form der Trennung aufgezeichnet, bei der die erfindungsgemäße Vorrichtung sich selbst ebenfalls vom sicheren Bereich 11a des Kommunikationsnetzes 11 trennen kann.

Das medizinische Gerät 10 kann dabei ggf. als System mehrerer medizinischer Teilgeräte 15, 16, die an einem sicheren Bereich 11a des Kommunikationsnetzes 11 angeschlossen sind, ausgeführt sein.

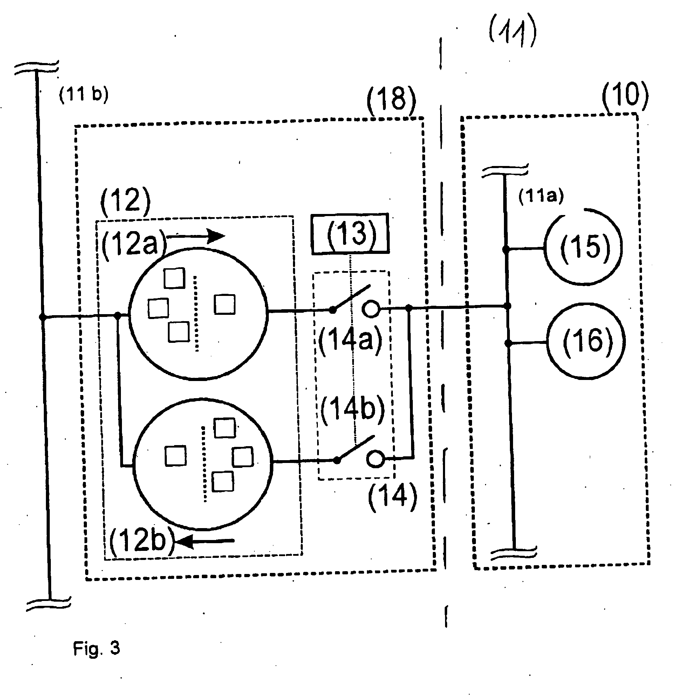

Die erfindungsgemäße Vorrichtung kann vorteilhaft ausgestaltet werden, indem die Paketfilterung bidirektional, also sowohl in Richtung vom unsicheren Bereich 11 b zum sicheren Bereich 11a des Kommunikationsnetzes 11 als auch in umgekehrter Richtung vorgenommen wird. In diesem Fall wird nicht nur das medizinische Gerät vor einer Attacke, die aus dem unsicheren Teil des Kommunikationsnetzes 11 erfolgen kann, abgesichert, sondern zusätzlich der Einfluss, den das medizinische Gerät im Fehlerfall auf diesen unsicheren Teil des Kommunikationsnetzes 11 ausübt, minimiert. In Fig. 3 ist die schematische Zeichnung einer erfindungsgemäßen Vorrichtung mit bidirektionaler Paketfilterung dargestellt.

Die erfindungsgemäße Vorrichtung mit bidirektionaler Paketfilterung 18 sichert ein medizinisches Gerät 10 gegen mögliche Attacken, die aus dem unsicheren Bereich 11 b des Kommunikationsnetzes 11 auftreten können, ab und sorgt gleichzeitig für eine im Fehlerfall minimale Belastung des unsicheren Teils 11 des Kommunikationsnetzes 11 durch das medizinische Gerät 10. Es bedient sich dazu eines Paketfilters 12, der sich aus zwei Paketfiltern zusammensetzt, die die verschiedenen Richtungen des Paketflusses überwachen. Auf dem Weg vom unsicheren Teil des Kommunikationsnetzes 11 zum medizinischen Gerät wird ein Paketfilter 12a eingesetzt, der Kommunikationspakete, welche das medizinische Gerät 10 potentiell gefährden können, nicht passieren lässt. In Gegenrichtung wird ein Paketfilter 12b eingesetzt, der die Rückwirkung des medizinischen Geräts auf den unsicheren Teil des Kommunikationsnetzes 11 minimieren kann. Eine Steuerungslogik 13 ermöglicht es, die Vorrichtung 9 selbst und den unsicheren Bereich 11 b des Kommunikationsnetzes 11 vom medizinischen Gerät 10 mit Hilfe des Unterbrechungsschalters 14 abzutrennen, wobei dieser sich aus zwei Teilen 14a und 14b für die unterschiedlichen Richtungen des Kommunikationsflusses zusammensetzt. Die beiden Teile des Unterbrechungsschalters werden dabei im Normalfall gleichzeitig betätigt, eine wechselweise Schaltung kann jedoch in Sonderfällen auch sinnvoll sein, z.B. zur Minimierung einer Auslastung des Kommunikationsnetzes 11.

Das medizinische Gerät 10 kann auch hier ggf. als System mehrerer medizinischer Teilgeräte 15, 16 die an dem sicheren Bereich 11a des Kommunikationsnetzes 11 angeschlossen sind, ausgeführt sein.

Eine durch den Unterbrechungsschalter 14 vorgenommene vollständige oder durch den Paketfilter 13 vorgenommene teilweise (da Kommunikationspakete ausbleiben) Trennung des medizinischen Geräts 10 vom unsicheren Bereich 11 b des Kommunikationsnetzes 11 muss im Rahmen der Risikoanalyse des medizinischen Geräts 10 betrachtet werden und zu einem sicheren Zustand desselben führen, in dem eine besondere Gefährdung des Bedieners oder Patienten des medizinischen Geräts 10 ausgeschlossen ist, um die erfindungsgemäße Vorrichtung 9 einsetzen zu können. In der Praxis dürfte diese Einschränkung des Gebrauchs der erfindungsgemäßen Vorrichtung 9 kaum relevant sein, da mit einem Abriss einer Netzwerkverbindung z.B. aufgrund von Fehlern in der Verkabelung immer gerechnet werden muss, und andere Verfahren zur Risikominimierung wie z.B. redundante Netzwerkarchitekturen in den meisten Einsatzfällen zu kostspielig sind.

Damit der Paketfilter 12 und der Unterbrechungsschalter 14 ihre Aufgaben wahrnehmen können, muss eine Segmentierung, also eine Trennung logischer und/oder physikalischer Art zwischen dem unsicheren Bereich 11 b und dem sicheren Bereich 11a des Kommunikationsnetzes 11 vorhanden sein. Anderenfalls könnten Kommunikationspakete ohne Zutun bzw. Möglichkeiten der Beeinflussung durch den Paketfilter 12 und Unterbrechungsschalter 14 beliebig zwischen unsicherem Bereich 11 b und sicherem Bereich 11a des Kommunikationsnetzes 11 transportiert werden.

Die Art und Weise, wie einer erfindungsgemäßen Vorrichtung eine Segmentierung der beiden Teile 11 und 17 des Kommunikationsnetzes 11 stattfindet, kann dabei völlig unterschiedlich sein. Als Beispiele für mögliche Segmentierungen bieten sich die Verwendung von auf anderen physikalischen Prinzipien beruhender Kommunikationsnetze 11 (z.B. LAN / WLAN), eines anderes Protokolls, der Transport von Dateien oder die Umsetzung von TCP/IP-Paketen an, es sind aber auch weitere Arten der Segmentierung denkbar.

Die Funktionsweise des Unterbrechungsschalters 14 hängt essentiell von der Art der verwendeten Segmentierung zwischen unsicherem Bereich 11 b und sicherem Bereich 11a des Kommunikationsnetzes 11 ab.

Wird eine erfindungsgemäße Vorrichtung mit bidirektionaler Paketfilterung 18 eingesetzt, kann die Segmentierung zwischen dem sicheren Bereich 11a und dem unsicheren Bereich 11 b des Kommunikationsnetzes 11 logisch erfolgen, z.B. indem hinter der erfindungsgemäßen Vorrichtung 18 ein anderes Kommunikationsprotokoll auf dem Kommunikationsnetz 11 verwendet wird, als vor der erfindungsgemäßen Vorrichtung. Im Normalfall nimmt der Paketfilter 12 - der die Protokollanalyse sowieso vornimmt - auch eine Übersetzung zwischen den verschiedenen Kommunikationsprotokollen vor.

Der Paketfilter 12a kann dann z.B. bei Bedarf die Protokollübersetzung für auszufilternde Kommunikationspakete nicht vornehmen. Damit werden z.B. im sicheren Bereich 11a des Kommunikationsnetzes 11 für die entsprechenden Kommunikationspakete keine gültigen übersetzten Kommunikationspakete mehr erzeugt. Es ist sogar vorstellbar, dass in diesem Fall die Funktion des Unterbrechungsschalters 14 durch den Paketfilter 12a mit übernommen wird, indem dieser die Übersetzung dauerhaft und für alle Kommunikationspakete einstellt. Das medizinische Gerät 10 ist dann logisch vom unsicheren Bereich 11 b des Kommunikationsnetzes 11 getrennt, wenn auch eine physikalische Trennung nicht erfolgt ist. In Gegenrichtung muss der Paketfilter 12b die entsprechenden Aufgaben übernehmen, und es gelten die gleichen Prinzipien in umgekehrter Richtung. Auf diese Weise lässt sich ein sehr kostengünstiger Aufbau der erfindungsgemäßen Vorrichtung realisieren.

Theoretisch ist es jedoch vorstellbar, dass Kommunikationspakete des im unsicheren Bereich 11 b des Kommunikationsnetzes 11 verwendeten Kommunikationsprotokolls auch im Kommunikationsprotokoll, welches im sicheren Bereich 11a des Kommunikationsnetzes 11 verwendet wird, gültige Kommunikationspakete darstellen, die eine Attacke auf das medizinische Gerät 10 durchführen. Zwar lässt sich dieses Risiko durch sorgfältige Auswahl der verwendeten Kommunikationsprotokolle minimieren, ein gewisses Restrisiko bleibt jedoch bestehen, wenn ein Angriff auf den sicheren Bereich 11a des Kommunikationsnetzes 11 durch einen ,Brut-Force-Angriff' oder einen ,Bubbling Idiot', der zufällige Kommunikationspakete sendet, erfolgt. In diesem Fall können nach einer gewissen Zeit gültige Kommunikationssequenzen für das Kommunikationsprotokoll, das auf dem sicheren Bereich 11a des Kommunikationsnetzes 11 eingesetzt wird, 'erraten' werden.

Daher kann ein besonders hohes Maß an Absicherung des medizinischen Geräts 10 erfolgen, wenn eine physikalische Segmentierung zwischen dem unsicheren Bereich 11b und dem sicheren Bereich 11a des Kommunikationsnetzes 11 besteht.

Eine solche physikalische Segmentierung kann realisiert werden, indem unterschiedliche physikalische Übertragungswege für den unsicheren Bereich 11 b und den sicheren Bereich 11a des Kommunikationsnetzes 11 eingesetzt werden. So könnte der unsichere Bereich 11 b des Kommunikationsnetzes 11 eine funkbasierte Übertragung, der sichere Bereich 11a eine Übertragung über Netzwerkkabel verwenden. Es lassen sich aber auch unterschiedliche Instanzen des gleichen Übertragungswegs nutzen, indem z.B. zwei getrennte Netzwerkverkabelungen oder unterschiedliche Funkträgerfrequenzen für die beiden Bereiche 11 a und 11 b des Kommunikationsnetzes 11 zur Verwendung kommen.

Eine besonders vorteilhafte Anwendung des beschriebenen Geräts 9 ergibt sich bei kabelgebundenen elektrischen Kommunikationsnetzen 11 jedoch, wenn eine physikalische Segmentierung der beiden Bereiche 11a und 11b des Kommunikationsnetzes 11 vorgenommen wird. In diesem Fall kann die beschriebene Vorrichtung nämlich auch eine Absicherung der medizinischen Geräte gegen elektrische Störungen, die durch den unsicheren Bereich 11 b des Kommunikationsnetzes 11 in das medizinische Gerät eingebracht werden können, und eine Fehlfunktion desselben und damit eine Gefährdung von Patient oder Anwender auslösen können.

Auch das für medizinische Geräte wichtige Absichern von Berührspannungen gegenüber fehlerhaften hohen Spannungen im unsicheren Bereich 11 b des Kommunikationsnetzes 11 kann in diesem Fall durch die erfindungsgemäße Vorrichtung 9 erfolgen.

Ist eine Absicherung der Berührspannungen bei der gegebenen Anwendung besonders kritisch, kann es vorteilhaft sein, für den sicheren Bereich 11a des Kommunikationsnetzes 11 ein auf Lichtwellenleitern basierendes Kommunikationsnetz 11 zu verwenden, da durch dieses prinzipiell keine Potentialausgleiche erfolgen.

Sowohl der unsichere Bereich 11 b des Kommunikationsnetzes 11 als auch der sichere Bereich 11a des Kommunikationsnetzes 11 können kabelgebunden sein, es sind aber auch andere Realisierungen denkbar, die die Daten über Licht, Funk, Schall oder andere Transportwege übermitteln. Die Methoden der Übertragung müssen in beiden Bereichen 11a und 11b des Kommunikationsnetzes 11 nicht die gleichen sein; in diesem Fall ist es vorteilhaft, wenn die erfindungsgemäße Vorrichtung eine Umsetzung zwischen diesen Übertragungsmethoden selbst vornehmen kann, da so nicht ein weiteres Gerät für diese Umsetzung benötigt wird.

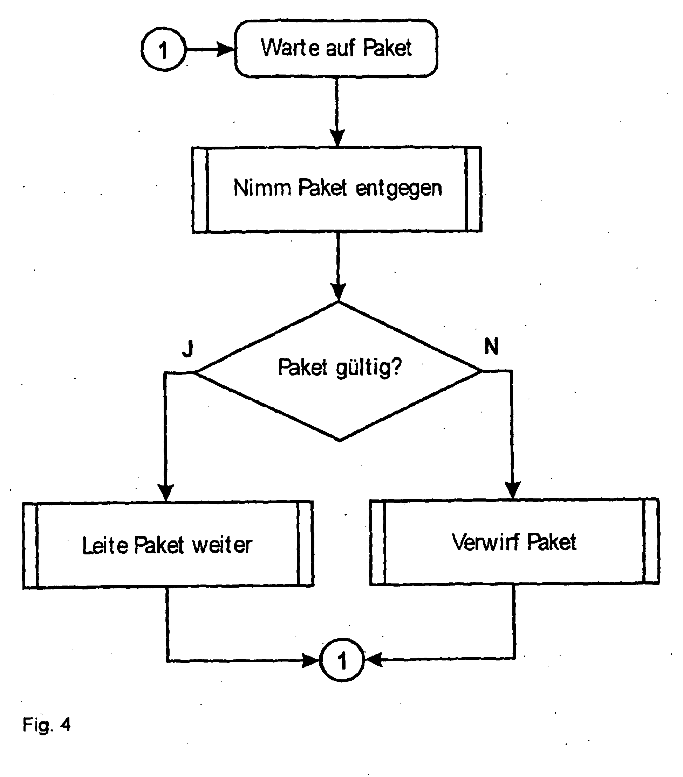

In Fig. 4 ist ein einfacher möglicher Grundalgorithmus für den Paketfilter 12 dargestellt. Der Paketfilter wartet auf den Empfang von Kommunikationspaketen aus dem unsicheren Bereich 11 b des Kommunikationsnetzes 11. Sobald ein Kommunikationspaket vorhanden ist, nimmt er dieses entgegen und prüft sodann, ob das Paket für die Weiterleitung an das medizinische Gerät 10 gültig ist. Ist dies der Fall, wird das Kommunikationspaket weitergeleitet, anderenfalls wird es verworfen. Anschließend wartet der Paketfilter 12 wieder auf den Empfang des nächsten Pakets.

Es kann hierbei insbesondere für eine Nachverfolgbarkeit des Verhaltens der erfindungsgemäßen Vorrichtung 9 von Vorteil sein, wenn ein Logging über empfangene und verworfene Kommunikationspakete durchgeführt werden und damit die korrekte Konfiguration des Paketfilters 12 geprüft werden kann.

Um entscheiden zu können, welche Kommunikationspakete vom unsicheren Bereich 11b des Kommunikationsnetzes 11 in den sicheren Bereich 11a des Kommunikationsnetzes 11 übernommen werden sollen, wird vom Paketfilter 12 zusätzliches Wissen über die Struktur der medizinischen Einrichtung, der inneren Struktur des medizinischen Geräts und seiner Subgeräte, deren Eigenschaften, momentaner Zustand und Ähnliches ausgewertet.

Im einfachsten Fall könnten z.B. lediglich die von festen MAC-Adressen oder IP-Adressen stammenden Kommunikationspakete den Paketfilter 12 passieren, oder nur solche, die die Kommunikation auf erlaubten Ports durchführen. Auch weitergehende Prüfungen der Kommunikation, wie eine Stateful Inspection können durchgeführt werden. Das Verhalten der erfindungsgemäßen Vorrichtung 9 wäre auf der Stufe der Überwachung der Netzwerkverbindungen in diesem Fall ähnlich zu demjenigen einer Hardware-Firewall; die Regeln zur Paketfilterung könnten jedoch wesentlich genauer spezifiziert und dem zu schützenden medizinischen Gerät angepasst werden. Im Unterschied zu gewöhnlichen Firewalls wird hier jedoch bei Feststellung gefährdender Kommunikationspakete die Verbindung zum unsicheren Bereich 11 b des Netzwerks 11 getrennt.

Als besonders vorteilhaft stellt es sich zudem dar, wenn die erfindungsgemäße Vorrichtung 9 eine Protokollanalyse des an das medizinische Gerät 10 zu übertragenden Kommunikationsprotokolls durchführt, und nur solche Kommunikationspakete den Paketfilter 12 passieren lässt, die dem Kommunikationsprotokoll des medizinischen Geräts 10 entsprechen. Dadurch ist die Gefahr einer Attacke auf das medizinische Gerät 10 bereits deutlich reduziert.

Darüber hinaus ermöglicht es eine Prokollanalyse auch, im Rahmen des Protokolls auftretende Daten auf gültige Datenwerte zu überprüfen und nur solche Kommunikationspakete den Paketfilter 12 passieren zu lassen, deren Daten im erlaubten Wertebereich sind.

Im Gegensatz zu Firewalls, welche Kommunikationsnetze 11 für allgemein verwendbare Computersysteme absichern, kann der Paketfilter 12 nämlich genau auf die zu verwendenden Kommunikationsprotokolle angepasst werden, da die Installation beliebiger Programme auf den in medizinischen Geräten eingebetteten Computersystemen im Allgemeinen weder möglich noch vom Hersteller zugelassen ist, wenn von diesen Geräten eine mögliche Gefährdung für den Bediener oder Patienten des medizinischen Geräts ausgeht. Deshalb erfahren die von einem medizinischen Gerät verwendeten Kommunikationsprotokolle seltener Änderungen, die ggf. einer Anpassung des Paketfilters bedürfen, als dies bei allgemein verwendbaren Computersystemen der Fall ist.

Eine besonders vorteilhafte Ausprägung der erfindungsgemäßen Vorrichtung 18 mit bidirektionaler Paketfilterung ergibt sich dann, wenn eine stattfindende Prüfung der eingesetzten Kommunikationsprotokolle auch zugleich genutzt wird, um die Daten in von anderen medizinischen Geräten verwendete Kommunikationsprotokolle und/oder in ein auf anderen physikalischen Prinzipien beruhendes Kommunikationsnetz 11 zu übersetzen und damit eigentlich inkompatiblen Geräten die Kommunikation miteinander zu ermöglichen.

Auch um entscheiden zu können, wann eine Trennung vom potentiell unsicheren Teil des Kommunikationsnetzes 11 vorgenommen werden soll, wird zusätzliches Wissen über die Struktur der medizinischen Einrichtung, der inneren Struktur des oder der medizinischen Geräte, deren Eigenschaften und Ähnliches ausgewertet.

Außerdem kann die beschriebene Vorrichtung im Falle einer erkannten potentiellen Gefahr durch den unsicheren Bereich 11 b des Kommunikationsnetzes 11 die Verbindung desselben zum medizinischen Gerät 10 vollständig unterbrechen, sodass eine Einschränkung mancher seiner Funktionen möglich ist, seine Kernfunktionalität jedoch noch gegeben ist.

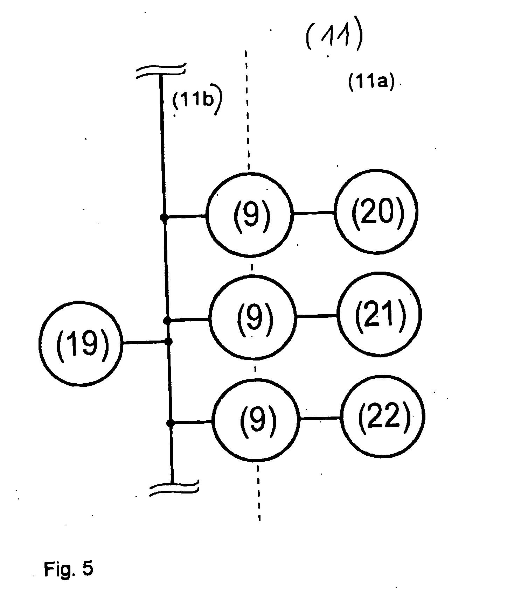

Am Beispiel eines Patientenmonitor-Systems, das schematisch in Fig. 5 dargestellt ist, soll eine derartige Abtrennung verdeutlicht werden. Eine Zentrale 19, die im unsicheren Bereich 11b des Kommunikationsnetzes 11 liegt, ist mit mehreren durch erfindungsgemäße Vorrichtungen 9 abgesicherten Patientenmonitoren 20, 21 und 22 verbunden, die damit jedes für sich in einem sicheren Bereich 11a des Kommunikationsnetzwerks 11 liegen. Im Fall einer erkannten potentiellen Gefahr kann durch die erfindungsgemäße Vorrichtungen 9 die Verbindung zum unsicheren Bereich 11 b des Kommunikationsnetzes 11 und damit zur Zentrale 19 unterbrochen werden, was eine Einschränkung der Funktionalität der Patientenmonitore 20, 21 und 22 darstellt, deren Kernfunktionalität, das Monitoring - wenn auch weniger komfortabel - dennoch weiterhin zur Verfügung steht. In der Zeit der Unterbrechung können keinerlei Zugriffe von den Patientenmonitoren 20, 21 und 22 auf die Zentrale 19 und umgekehrt durchgeführt werden.

Eine solche Trennung kann insbesondere dann sehr vorteilhaft sein, wenn z.B. ältere medizinische Geräte eingesetzt werden, die nur unzulänglich für die auf heutigen Kommunikationsnetzen 11 hohen Datenraten vorbereitet sind, da bei der Entwicklung des Geräts von dedizierten Kommunikationsnetzwerken 11 für eben diese Geräte ausgegangen wurde, diese Voraussetzung aber aufgrund der Infrastruktur der medizinischen Einrichtung nicht mehr gegeben ist. Sind z. B. aufwändige Interruptbehandlungsroutinen in den medizinischen Geräten eingesetzt, kann u.U. bereits eine reine Überlast auf dem Kommunikationsnetz 11 dazu führen, dass eine unzulässig hohe Auslastung der Prozessoren in den medizinischen Geräten hervorgerufen wird, selbst wenn die eigentlichen übertragenen Daten völlig harmlos sind. Während sich ein einzelnes medizinisches Gerät in diesem Fall in einen sicheren Zustand begeben muss, kann der gleichzeitige Übergang einer größeren Gruppe medizinischer Geräte in genau diesen Zustand einen durch die Gruppenkomplexität hervorgerufenen kritischen Gesamtzustand hervorrufen. Wenn also zum Beispiel ein einzelner Patientenmonitor in einer Intensivstation einen Fehler aufweist, diesen aber selbständig erkennt und mit einer Alarmgabe den Dienst mit einem Gerätealarm einstellt, ist diese Situation per se nicht unbedingt als kritisch einzustufen. Ähnliches gilt für eine Infusionspumpe. Fallen aber auf der selben Intensivstation aufgrund von Störungen, die durch das Kommunikationsnetz 11 provoziert werden, alle Patientenmonitore und alle Infusionspumpen gleichzeitig aus, ist die entstehende Gefährdung für alle dort behandelten Patienten ungleich größer.

Die Steuerungslogik 13 kann die Entscheidung über das Durchführen einer Trennung der Bereiche des Kommunikationsnetzes 11 anhand von statischen Prüfungen der zu übermittelnden Daten vornehmen. Als solche bieten sich z.B. eine Prüfung von IP-Adressen der Kommunikationspartner, der verwendeten MAC-Adressen dieser, eine Verwendung bestimmter Ports und/oder insbesondere eine Syntax- und/oder Semantik- und Konsistenzprüfung der übermittelten Daten an.

Im Normalfall wird eine statische Prüfung von zu übermittelnden Kommunikationspaketen eher zu einem Verwerfen der Pakete im Paketfilter als zu einer Trennung von unsicheren Bereich 11 b des Kommunikationsnetzes 11 führen. Im Falle besonders hoher Sicherheitsanforderungen für das medizinische Gerät 10 kann es jedoch sinnvoll sein, wenn zu häufige Verletzungen der statischen Gültigkeitsprüfungen zu einer temporären oder sogar dauerhaften Trennung vom unsicheren Bereich 11 b des Kommunikationsnetzes 11 führen.

Eine Entscheidung über das Durchführen einer Trennung der Bereiche 11 a und 11 b des Kommunikationsnetzes 11 kann aber auch erfolgen, indem eine dynamische Prüfung der zu übermittelnden Daten durchgeführt wird. Solche Prüfungen können z.B. die Antwortzeiten des Kommunikationspartners, ein maximaler zulässiger Datenfluss zu den zu schützenden medizinischen Geräten oder auch ein minimaler Datenfluss, der auf eine korrekte Funktion des Kommunikationspartners hinweist, sein. Auch zustandsbasierte Überwachungen, die ein Modell der an der Kommunikation beteiligten Geräte beinhalten, sind hier sehr vorteilhaft.

Eine besondere Form der Nutzung des erfindungsgemäßen Geräts 9 ergibt sich, wenn mehrere medizinische Subgeräte, die gemeinsam als ein medizinisches System/gerät zusammenarbeiten, durch einen unsicheren Teil 11 b des Kommunikationsnetzes 11 miteinander verbunden werden. In dieser Konstellation empfiehlt es sich, die medizinischen (Sub-)Geräte jeweils einzeln mit erfindungsgemäßen Vorrichtungen 9 auszustatten. Es kann in diesem Fall sogar sinnvoller sein, erfindungsgemäße Vorrichtungen 18 mit bidirektionaler Paketfilterung zu verwenden, wie später dargelegt wird.

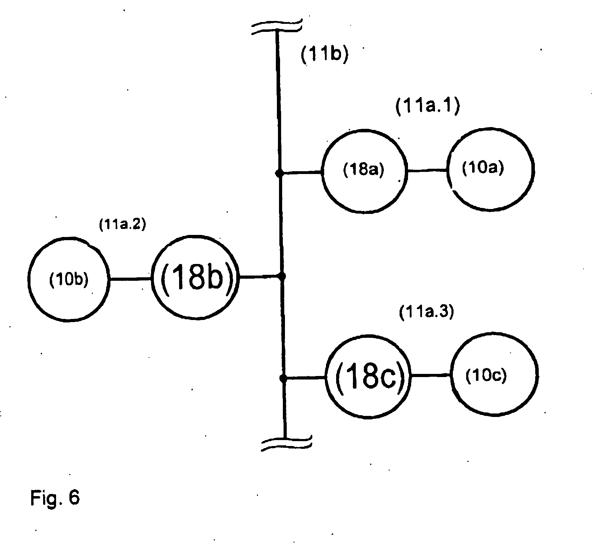

In Fig. 6 ist der Aufbau eines Systems medizinischer Geräte, welche durch einen unsicheren Teil des Kommunikationsnetzes 11 miteinander verbunden sind, dargestellt. Über den unsicheren Bereich 11 b des Kommunikationsnetzes 11 sind die beiden medizinischen Geräte 10a und 10b, welche zusammen ein medizinisches System bilden, miteinander verbunden. Sie hängen an jeweils durch die erfindungsgemäßen Vorrichtungen mit bidirektionaler Paketfilterung 18a und 18b abgesicherten sicheren Bereichen 11 a.1 und 11 b.2 des Kommunikationsnetzes 11. Ein weiteres, jedoch nicht oben genanntem medizinischen System zugehöriges medizinisches Gerät 10c ist durch die ihm zugeordnete erfindungsgemäße Vorrichtung 18c abgesichert und mit dem unsicheren Bereich 11 b des Kommunikationsnetzes 11 verbunden.

Sind die Paketfilter der erfindungsgemäßen Vorrichtungen 18a, 18b und 18c optimal eingestellt und werden geeignete Kommunikationsprotokoll verwendet, kann zwischen den medizinischen Geräten 10a und 10b eine Kommunikation stattfinden, ohne dass diese vom medizinischen Gerät 10c bemerkt wird. Umgekehrt kann zwischen dem medizinischen Gerät 10c und anderen, sich im unsicheren Bereich des Kommunikationsnetzes 11 befindlichen Geräten eine Kommunikation stattfinden, ohne dass diese die medizinischen Geräte 10a und 10b beeinflusst. Wenn aber die von den Geräten 10a, 10b und 10c verwendeten Kommunikationsprotokolle gleich sind, und z.B. darüber hinaus ein Anteil der Kommunikation als Broadcast - also alle Teilnehmer im Kommunikationsnetz 11 gleichzeitig ansprechend - durchgeführt wird, kann die oben genannte Methode versagen.

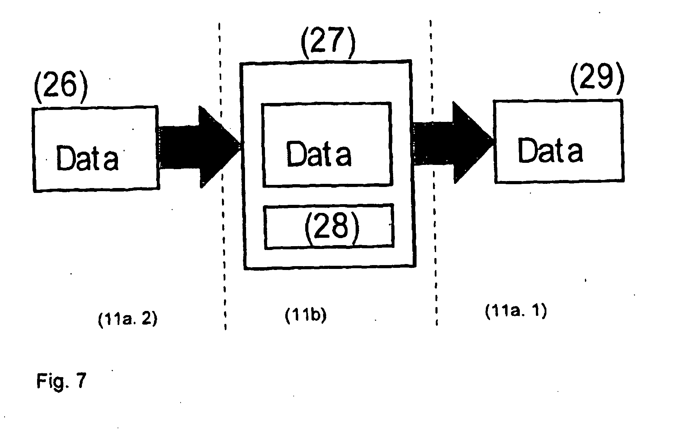

In diesem Fall empfiehlt es sich, wenn die Paketfilter 13 der den medizinischen Geräten 18a und 18b zugeordneten erfindungsgemäßen Vorrichtungen 10a und 10b eine Übersetzung der Kommunikationsprotokolle so vornehmen, dass im unsicheren Bereich 11 b des Kommunikationsnetzes 11 gegenüber dem Originalprotokoll modifizierte Kommunikationspakete verwendet werden. In Fig. 7 ist der Ablauf einer solchen Vorgehensweise unter Bezug auf Fig. 6 schematisch dargestellt.

Ein Kommunikationspaket 26, welches die als ,Data' gekennzeichneten Daten enthält wird von der erfindungsgemäßen Vorrichtung 18b beim Übergang vom einen sicheren Bereich 11a.2 des Kommunikationsnetzes 11 in ein anderes Kommunikationspaket 27, welches innerhalb des unsicheren Bereich 11 b des Kommunikationsnetzes 11 transportiert wird, umgewandelt. Die Umwandlung kann dabei sehr einfach und schnell erfolgen, indem das originale Kommunikationspaket 26 in ein anderes Kommunikationspaket 27 eingebettet wird. Es kann dabei vorteilhaft sein, wenn zusätzliche Daten 28 - wie z.B. eine Signatur-in das Kommunikationspaket 27 eingebettet sind, z.B. um bei gleichen Protokollen verschiedene Gruppen von medizinischen Geräten voneinander zu trennen und/oder um als erfindungsgemäße Vorrichtung 18a feststellen zu können, dass die Daten innerhalb des unsicheren Teils 11 b des Kommunikationsnetzes 11 nicht verändert wurden. Das erfindungsgemäße Gerät 18a wandelt bei einer Weiterleitung das modifizierte Kommunikationspaket 27 in ein vom medizinischen Gerät 10a verwendbares Kommunikationspaket 29 um, welches im bei gleichem verwendeten Kommunikationsprotokoll meist identisch zum ursprünglichen Kommunikationspaket 26 sein dürfte.

Auf diese Weise ist es effizient möglich, die wechselseitige Beeinflussung verschiedener Kommunikation auf ein Mindestmaß zu reduzieren. Wird vom erfindungsgemäßen Gerät 18a z.B. durch eine Signaturprüfung festgestellt, dass das ankommende Kommunikationspaket 27 eine Verfälschung erfahren hat, wird das entsprechende Paket 29 nicht erzeugt. Es besteht dann evtl. ein Indiz für eine Attacke und die Steuerungslogik 13 könnte die Trennung vom unsicheren Teil des Netzes - ggf. auch erst nach häufigerem Auftreten solcher Vorfälle - durchführen.

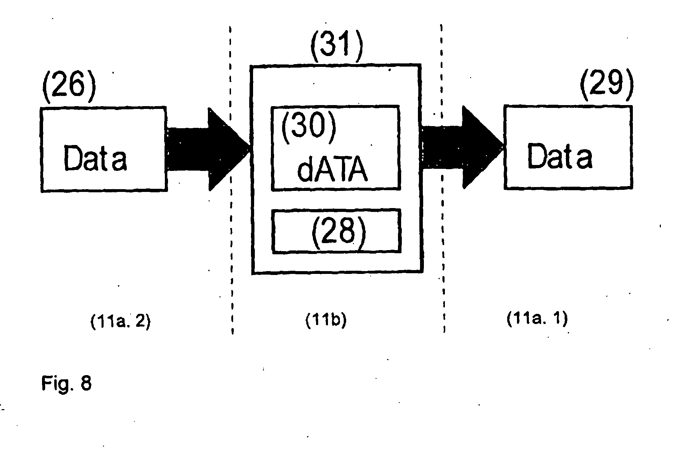

Bei der beschriebenen Netzstruktur, bei der der unsichere Bereich 11 b des Kommunikationsnetzes 11 verwendet wird, um zwei medizinische Subgeräte miteinander kommunizieren zu lassen, können jedoch neben den zuvor beschriebenen potentiellen Gefährdungen von Patient und/oder Bediener auch Anforderungen an die Sicherung von Daten gegenüber Einsichtnahme durch unbefugte Dritte gestellt werden. Es kann durch eine Erweiterung der in Fig. 7 gezeigten Vorgehensweise diese Anforderung erfüllt werden. In Fig. 8 ist der Ablauf dieser erweiterten Vorgehensweise dargestellt.

Ein Kommunikationspaket 26 enthält die als ,Data' gekennzeichneten Daten. Es wird von der erfindungsgemäßen Vorrichtung 18b beim Übergang vom einen sicheren Teil 11a.1 des Kommunikationsnetzes 11 in den unsicheren Bereich 11b in ein anderes modifiziertes Kommunikationspaket 31 umgewandelt. Im Gegensatz zum Vorgehen in Fig. 7 wird das ursprüngliche Kommunikationspaket nicht einfach in das neue Kommunikationspaket eingebettet, sondern der Datenbereich in einen verschlüsselten Datenbereich 30, der als 'dATA' gekennzeichnet ist, umgewandelt. Dabei können die aus anderen Anwendungsbereichen symmetrischen und/oder unsymmetrischen Verfahren eingesetzt werden, ggf. mit einer zusätzlich eingebetteten Signatur zur Unterscheidung verschiedener Gruppen medizinischer Geräte oder schneller Prüfung der Unversehrtheit der Kommunikationspakete. Beim Übergang in den zweiten sicheren Teil 11a.2 des Kommunikationsnetzes 11 wird das modifizierte und verschlüsselte Kommunikationspaket 31 in ein vom medizinischen Gerät 23 verwendbares Kommunikationspaket 29 umgewandelt, welches bei gleichem verwendeten Kommunikationsprotokoll meist identisch zum ursprünglichen Kommunikationspaket 26 ist.

Es entsteht dadurch eine Art virtuelles privates Netzwerk (VPN) zwischen den Geräten 10a und 10b sodass Inhalte der Kommunikation von anderen Kommunikationsfähigen Geräten im unsicheren Bereich 11 b des Kommunikationsnetzes 11 weder unbemerkt modifiziert noch ausgelesen werden können. Besonders vorteilhaft stellt sich diese Vorgehensweise dar, wenn es sich beim unsicheren Bereich 11 b des Kommunikationsnetzes 11 um das Internet handelt, wie es bei der Anbindung eines medizinischen Geräts in der Wohnung des Patienten meist der Fall sein dürfte.

Diese Sicherung kann sehr vorteilhaft sogar innerhalb einer medizinischen Einrichtung eingesetzt werden, um Daten durch einen ,öffentlicheren Teil' des Kommunikationsnetzes 11 zu transportieren, ohne Unbefugten die Möglichkeit zu geben, die Daten einzusehen. Wenn eine sehr detaillierte Analyse der übertragenen Daten zur Absicherung durchgeführt wird, kann die beschriebene Erfindung besonders effizient eine Auftrennung der Daten in einen unverschlüsselten ,öffentlichen' und einen verschlüsselten 'privaten' Teil vornehmen.

Sollen Daten zwischen medizinischen Subgeräten 10a und 10b ausgetauscht werden, die ein besonders hohes Gefährdungspotential bei einer möglichen Verfälschung aufweisen, kann es von Vorteil sein, eine solche potentielle Verfälschung im unsicheren Bereich 11 b des Kommunikationsnetzes 11 durch das Speichern und Senden ,veralteter' Kommunikationspakete zu verhindern, indem in jedem über den unsicheren Bereich 11 b des Kommunikationsnetzes 11 gesendeten Kommunikationspaket ein veränderlicher Teil enthalten ist, dessen Korrektheit beim Empfang geprüft wird, bevor das Kommunikationspaket den Paketfilter passiert. Dieser kann z.B. eine einfache Zahl mit einer definierten Folge sein. Wird als veränderlicher Teil eine Uhrzeit verwendet, kann sogar geprüft werden, ob das Paket noch gültig ist, oder bereits zu viel Zeit für den Transport im Kommunikationsnetz 11 benötigt hat.

Wenn eines der medizinischen Subgeräte 10a und 10b bei einem Abreißen der Kommunikation zum jeweils anderen medizinischen Subgerät eine besondere Reaktion erfordert, die Kommunikation jedoch keinem festen Zeitraster gehorcht, können die zugehörigen erfindungsgemäßen Vorrichtungen 18a und 18b in festen Zeitabständen Lebenszeichen senden, um die Funktionsweise der Kommunikation überprüfen zu können. Beim Ausbleiben dieser Lebenszeichen muss dann das entsprechende zu überwachende Gerät 10a bzw. 10b auf geeignete Weise benachrichtigt werden. Dies kann z.B. über die Generierung eines geeigneten Kommunikationspakets oder auch über die Verwendung dedizierter Steuerleitungen geschehen.

Im Falle noch höherer Anforderungen an eine Manipulationssicherheit zu übertragender Daten kann von der sendenden erfindungsgemäßen Vorrichtung 18a oder 18b zu jedem relevanten Kommunikationspaket eine Transaktionsnummer (TAN) mit übermittelt werden, anhand derer die empfangene erfindungsgemäße Vorrichtung 18b bzw. 18a die Herkunft des Kommunikationspakets zweifelsfrei bestimmen kann. Eine Liste von TANs muss dann beiden erfindungsgemäßen Vorrichtungen 18a und 18b vorher auf anderem, sicherem Weg - z.B. durch Datenträger- übermittelt worden sein. Mit jeder an den jeweiligen Kommunikationspartner übermittelten TAN wird diese von beiden erfindungsgemäßen Vorrichtungen 18a und 18b aus der Liste gestrichen.

Eine zusätzliche Form des Verbergens von Daten, die in den unsicheren Bereich 11 b des Kommunikationsnetzes 11 übermittelt werden, kann auch erfolgen, indem Adressierungen im geschützten Teil des Kommunikationsnetzes 11 nicht nach außen bekannt gegeben werden. Dies erschwert mögliche Beeinflussungen aus dem potentiell unsicheren Kommunikationsnetzwerk 11 deutlich.

Aufgrund der Funktionsweise der beschriebenen Vorrichtung kann dieses sehr vorteilhaft auch weitere Aufgaben übernehmen, die bisher von speziellen Geräten wahrgenommen werden. So ist es ohne erheblichen zusätzlichen Aufwand möglich, einen Virenscanner in das Gerät zu integrieren, der den gesamten Datenverkehr auf das Vorhandensein von Computerviren überprüft.

Die beschriebene Vorrichtung kann auch ein Caching von über das Kommunikationsprotokoll zu übertragenden Daten vornehmen, diese immer oder nur für die Dauer einer Abtrennung vom potentiell unsicheren Bereich 11 b des Kommunikationsnetzes 11 zwischenspeichern, um sie nach einem erfolgreichen Wiederaufbau der Verbindung selbständig an den Kommunikationspartner zu übertragen.

Sie kann ein Proxying vornehmen, und so z.B. einem Gerät, das ein Gerät an einer seriellen Schnittstelle mit den entsprechend schnellen Antwortzeiten angeschlossen werden soll, bei einer Protokollübersetzung und Verbindung über das Internet mit entsprechend langsamen Antwortzeichen Bestätigungen für den Empfang von Paketen zunächst pauschal zurücksenden, und erst beim längeren Ausbleiben dieser Signale das zu schützende Gerät in einen Modus setzen, in dem die Verbindung als aufgetrennt betrachtet wird.

Andere mögliche zusätzliche Aufgaben, die vorteilhaft von dem beschriebenen Gerät erledigt werden können, sind z. B. eine Zugangskontrolle für im gesicherten Kommunikationsnetzwerk 11 liegenden Daten, oder sogar den Schutz von SW-Lizenzen im Kommunikationsnetzwerk 11.

Durch den direkten Kontakt zum unsicheren Bereich 11 b des Kommunikationsnetzwerks 11 besteht jedoch die Gefahr, dass das beschriebene Gerät selbst durch eine Attacke aus dem Kommunikationsnetzwerk 11 in seiner Funktionsweise gestört wird, und daher seine Schutzfunktion nicht oder nur noch eingeschränkt wahrnehmen kann.

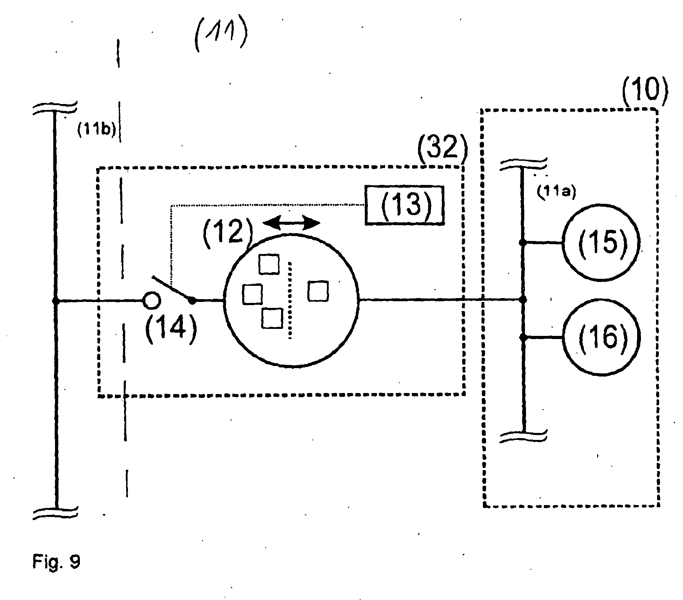

Es kann daher sinnvoll sein, wenn die erfindungsgemäße Vorrichtung sich selbst mit Hilfe des Unterbrechungsschalters vom unsicheren Bereich 11 b des Kommunikationsnetzes 11 abtrennen kann, wenn die Steuerungslogik 13 eine potentielle Attacke auf das medizinische Gerät oder das erfindungsgemäße Gerät feststellt. In Fig. 9 ist der schematische Aufbau einer derart gestalteten erfindungsgemäßen Vorrichtung gezeigt.

Beispielhaft ist hier die Paketfilterung 12 bidirektional ausgelegt; es ist jedoch auch eine unidirektionale Paketfilterung, wie in Fig. 2 dargestellt, denkbar. Bei der erfindungsgemäßen Vorrichtung 32 befindet sich der Unterbrechungsschalter 14 auf der Seite, die an den unsicheren Bereich 11 b des Kommunikationsnetzes 11 angeschlossen ist. So kann die Steuerungslogik 13 das erfindungsgemäße Gerät 32 zusammen mit dem medizinischen Gerät 10 vom unsicheren Bereich 11 b des Kommunikationsnetzes 11 trennen.

Aufgrund der völligen Trennung vom unsicheren Bereich 11 b des Kommunikationsnetzes 11 ist eine weitere Attacke auf die erfindungsgemäßen Vorrichtung 32 ausgeschlossen. Der Zustand der Abtrennung vom unsicheren Bereich 11 b des Kommunikationsnetzes 11 muss den sicheren Zustand für das medizinische Gerät 10 darstellen und ist z.B. auch bei einem Ausfall der Versorgungsspannung des erfindungsgemäßen Geräts 32 einzunehmen.

Es kann eine sinnvolle Erweiterung des Konzepts sein, wenn die Sicherungslogik 13 die Möglichkeit hat, einen Reset und damit verbunden einen Neustart der erfindungsgemäßen Vorrichtung 32 vorzunehmen, damit sichergestellt ist, dass die Vorrichtung in einen definierten, korrekten Anfangszustand versetzt wird, nachdem eine potentielle Attacke auf die erfindungsgemäße Vorrichtung durch Öffnen des Unterbrechungsschalters 14 abgewehrt wurde.

Eine noch kritischere Situation könnte sich ergeben, wenn das erfindungsgemäße Gerät 9 durch eine Fehlfunktion selbst eine Attacke auf das zu schützende medizinische Gerät 10 durchführt. Es stellt sich daher, wie in Fig. 2 dargestellt, als vorteilhaft dar, wenn die Steuerungslogik 13 mit Hilfe des Unterbrechungsschalters 14 das erfindungsgemäße Vorrichtung 9 zusammen mit dem unsicheren Bereich 11 b des Kommunikationsnetzes 11 vom sicheren Bereich 11a des Kommunikationsnetzes 11 trennt, sobald eine Einschränkung des Funktionsweise des erfindungsgemäßen Geräts 9 festgestellt wird. Als sicherer Zustand der erfindungsgemäßen Vorrichtung 9 wird demgemäß eine Öffnung des Unterbrechungsschalters 14 und damit die Trennung des unsicheren Teils 11 und der erfindungsgemäßen Vorrichtung 9 vom sicheren Bereich 11a des Netzes verstanden. Dieser Zustand ist also z.B. auch bei einem Versagen der Versorgungsspannung der erfindungsgemäßen Vorrichtung 9 einzunehmen.

Um eine solche Einschränkung der eigenen Funktionsweise festzustellen, können, z.B. folgende Verfahren eingesetzt werden:
- Hard- oder Software-Watchdogs
- Logische und/oder zeitliche Programmlaufüberwachung der eingesetzten Software
- Stacküberwachung der eingesetzten Software
- Überprüfung der korrekten Funktionsweise von ROM und RAM Speicher
- Überwachung der Spannungsversorgung
- Prüfung auf Unversehrtheit von Programmcode und Daten

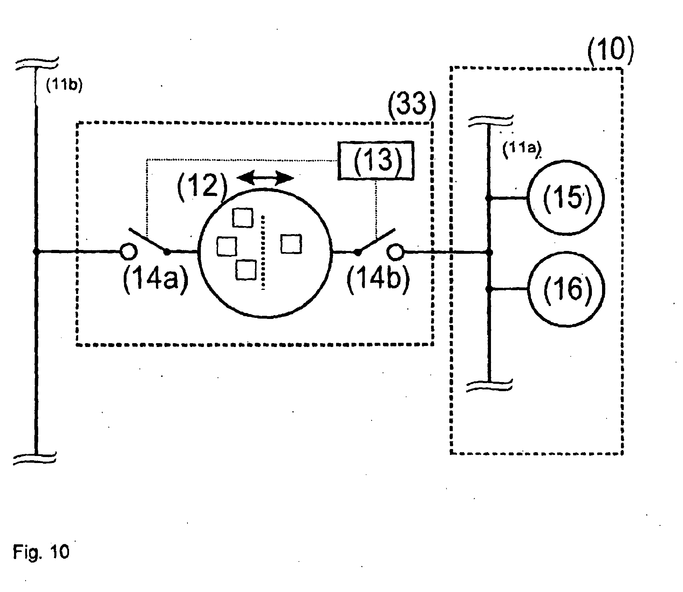

Da sowohl die geschilderte Struktur laut Fig. 2, als auch die Struktur laut Fig. 9 Vorteile bei der Realisierung der erfindungsgemäßen Vorrichtung bieten, erscheint es für besonders hohe Sicherheitsanforderungen sinnvoll, eine Kombination beider Strukturen, wie sie in Fig. 10 dargestellt ist, einzusetzen. In der dort dargestellten Konfiguration kann die Steuerungslogik 13 kann die Verbindung der erfindungsgemäßen Vorrichtung 33 sowohl zum unsicheren Bereich 11 b als auch zum sicheren Bereich 11a des Kommunikationsnetzes 11 mit Hilfe der Unterbrechungsschalter 14a und 14b trennen.

Dabei kann eine solche Trennung durch beide Unterbrechungsschalter entweder aufeinander abgestimmt oder unabhängig voneinander erfolgen. Als Unterstützung einer physikalischen Segmentierung der beiden Bereiche 11a und 11b des Kommunikationsnetzes 11 kann es sinnvoll sein, die Schalter 14a und 14b ein Schließen stets wechselweise durchführen zu lassen, da so die physikalische Segmentierung auch im Falle einer Fehlfunktion der erfindungsgemäßen Vorrichtung 33 zu jedem Zeitpunkt erhalten bleibt.

Im Falle einer von der Steuerungslogik festgestellten Funktionsunfähigkeit des Geräts 33 empfiehlt es sich, beide Unterbrechungsschalter 14a und 14b gleichzeitig zu öffnen, und einen Reset durchzuführen, um den nach einem Neustart vorhandenen definiert sicheren Zustand des erfindungsgemäßen Geräts 33 zu erzielen. Die Trennung von beiden Teilen des Kommunikationsnetzes 11 sollte auch den sicheren Zustand des medizinischen Geräts 10 darstellen und z.B. beim Ausbleiben der Versorgungsspannung des Geräts 33 von diesem eingenommen werden.

Eine im Sinne der Sicherheit besonders vorteilhafte Realisierung der erfindungsgemäßen Vorrichtung ergibt sich dann, wenn die erfindungsgemäße Vorrichtung redundant aufgebaut wird. Hierzu bieten sich eine Reihe von möglichen Verfahren an.

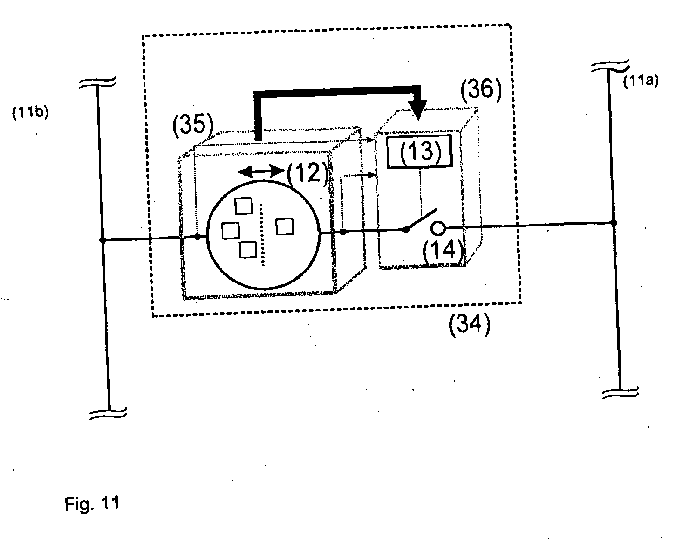

In Fig. 11 ist eine mögliche, einfache redundante Realisierung der erfindungsgemäßen Vorrichtung - der Einfachheit halber wieder beispielhaft mit einer bidirektionalen Paketfilterung - dargestellt.

Die erfindungsgemäße Vorrichtung 34 besteht hierbei aus einem Arbeitskanal 35 und einem Überwachungskanal 36, die in voneinander getrennten Subsystemen untergebracht sind. Der Arbeitskanal 35 übernimmt die Funktionen des Paketfilters 12 und alle u.U. damit verbundenen Funktionen, also z.B. auch eine Umsetzung der verwendeten Kommunikationsprotokolle. Der Überwachungskanal 36 nimmt diesbezüglich keine Tätigkeit vor. Seine Funktion besteht lediglich darin, die korrekte Funktionsweise des Arbeitskanals 35 zu überwachen. Dies kann geschehen, indem ein (vereinfachtes) Modell der Aufgabenstellung des Arbeitskanals im Überwachungskanal existiert, oder der Überwachungskanal 36 sogar nur nach einem Watchdog-Prinzip die Funktion des Arbeitskanals 35 prüft. Stellt der Überwachungskanal 36 einen Fehler des Arbeitskanals 35 fest, kann er diesen mit Hilfe des Unterbrechungsschalters 14 vom Kommunikationsnetz 11 trennen und bei ihm ggf. einen Reset auslösen, um ihn wieder in einen definierten und somit seine Funktion erfüllenden Zustand zu bringen.

Dabei ist in Fig. 11 beispielhaft eine Trennung auf der Seite des sicheren Teils 17 des Kommunikationsnetzes 11 dargestellt; es sind aber genauso auch Varianten mit einer Trennung vom unsicheren Bereich 11 b oder beiden Bereichen des Kommunikationsnetzes 11 möglich.

Eine sehr sichere Variante besteht in komplett voneinander unabhängigen ,diversitären' Kanälen, von denen jeder sich und den anderen Kanal vom potentiell unsicheren Teil des Kommunikationsnetzes 11 trennen und die korrekte Funktionsweise des Gegenüber überwachen kann.

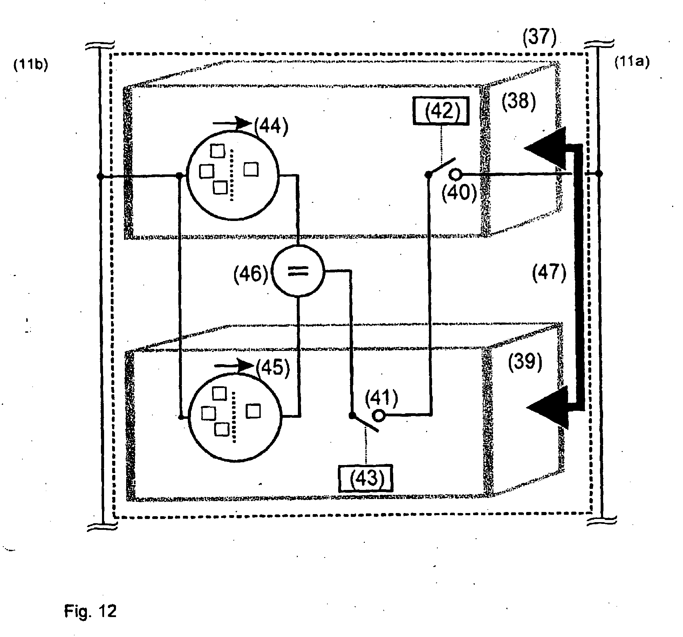

Eine solche Variante ist in Fig. 12 vereinfacht dargestellt. Der gesamte Teil, der Kommunikationspakete vom sicheren Bereich 11a in den unsicheren Bereich 11 b des Kommunikationsnetzes 11 transportiert, ist hierbei weggelassen. Er kann im Falle einer bidirektionalen Paketfilterung spiegelbildlich zum dargestellten Teil, sonst durch einfaches Weiterleiten der Kommunikationspakete realisiert werden. Auch hier erfolgt eine Trennung wieder beispielhaft auf der Seite des sicheren Bereich 11 a des Kommunikationsnetzes 11.

Die erfindungsgemäße Vorrichtung 37 besteht aus zwei voneinander unabhängigen Subsystemen 38 und 39, die jeweils einen Unterbrechungsschalter 40 bzw. 41, eine Steuerungslogik 42 bzw. 43 und einen Paketfilter 44 bzw. 45 enthalten. Sind die beiden Paketfilter 44 und 45 zu dem Ergebnis gekommen, ein Kommunikationspaket ggf. zu übersetzen und weiterzuleiten, werden die beiden Teilergebnisse von einem Vergleicher 46 geprüft und bei Gleichheit weitergeleitet. Die beiden Instanzen 42 und 43 der Steuerungslogik können jeweils unabhängig voneinander die Trennung der beiden Bereiche 11a und 11 b des Kommunikationsnetzes 11 vornehmen. Dabei können sie aus dem jeweils eigenen Subsystem 38 bzw. 39 stammende Informationen über möglicherweise stattfindende Attacken oder eine eingeschränkte Funktionalität des jeweiligen Subsystems auswerten. Besonders vorteilhaft ist es aber, wenn zwischen beiden den beiden Subsystemen 38 und 39 ein Kommunikationsweg 47 existiert mit dessen Hilfe die beiden Instanzen 42 und 43 der Steuerungslogik Hinweise auf die korrekte Funktionsweise des jeweils anderen Subsystems 39 bzw. 39 erhalten, damit sie eine Trennung der Teile 11 b und 11a des Kommunikationsnetzes 11 vornehmen können.

Auch in diesem Fall ist es von Vorteil, wenn die beiden Instanzen 42 und 43 der Steuerungslogik einen Reset des jeweils anderen Subsystems oder sogar der gesamten Vorrichtung 37 auslösen können.

Sollen zur Entwicklung der erfindungsgemäßen Vorrichtung 37 zum Beispiel Betriebssysteme eingesetzt werden, um Entwicklungszeit und Entwicklungsfehler zu minimieren, so empfiehlt es sich, in beiden Kanälen voneinander unabhängige Betriebssysteme und möglichst auch unterschiedliche Netzwerk-Hardware zu verwenden, da die Wahrscheinlichkeit, dass beide Kanäle gleichzeitig und gleichartig durch das potentiell unsichere Kommunikationsnetzwerk 11 beeinflusst werden, extrem gering und damit die Sicherheit gegen Fehlverhalten sehr hoch ist. Darüber hinaus bietet eine diversitäre Realisierung des beschriebenen Geräts auch den Vorteil, dass Fehler, die in Design und Entwicklung der Software und oder Hardware desselben entstanden oder durch zufällige Veränderung von Dateninhalten von Computerspeichern im Normalfall erkannt werden und zu einer bis zu einer Korrektur dauerhaften Abtrennung vom potentiell unsicheren Kommunikationsnetzwerk 11 führen.

Damit wird das Risiko minimiert, dass ein Fehler die Funktion des Geräts behindert, dieser aber bis zu einer möglichen Beeinflussung des medizinischen Geräts durch das potentiell unsichere Kommunikationsnetzwerk 11 noch nicht erkannt wird und die ein Patientenrisiko verursachende Beeinflussung dadurch tatsächlich stattfindet.

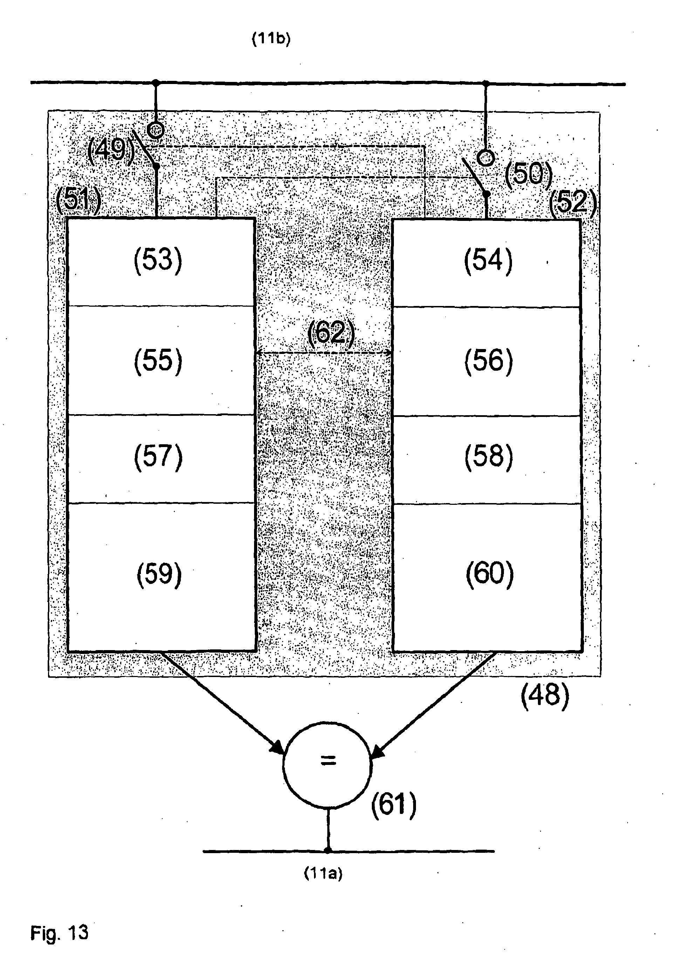

In Fig. 13 ist ein möglicher schematischer Aufbau einer solchen diversitären erfindungsgemäßen Vorrichtung 48 dargestellt; auch hier wieder nur für eine Transportrichtung von unsicheren Bereich 11b in den sicheren Bereich 11a des Kommunikationsnetzes 11.

Aus dem unsicheren Bereich 11 b des Kommunikationsnetzes 11 gelangen die Daten durch das Betriebsystem 53 in den ersten Kanal 51, von dort durchlaufen sie Firewall 55, einen Virenscanner 57 und die Protokollanalyse 59. Jede dieser Schichten verwirft die Daten, sobald diese potentiell gefährlich sind. Anschließend gelangen die Daten in den Vergleicher 61. Parallel dazu gelangen die Daten im zweiten Kanal 52 durch das zweite Betriebssystem 54, die zweite Firewall 56, den zweiten Virenscanner 58 und die zweite Protokollanalyse 60 ebenfalls in den Vergleicher 61. Lediglich für den Fall, dass beide Kanäle zum Ergebnis gekommen sind, dass die Daten korrekt und unschädlich sind, werden sie an den sicheren Bereich 11a des Kommunikationsnetzes 11 weitergeleitet. Die beiden Kanäle führen eine gegenseitige Überwachung 62 mit ggf. einem Auslösen von Reset durch und können sich gegenseitig über die Abschaltwege 49 und 50 vom unsicheren Bereich 11 b des Kommunikationsnetzes 11 abtrennen.

Mögliche Ausführungsformen des beschriebenen Geräts 9 können dabei als eigenständiges Gerät ebenso wie als in ein medizinisches Gerät eingebundenes Gerät sein. Eine Realisierung der erfindungsgemäßen Vorrichtung 9 wird in den meisten Fällen durch eine Kombination von Soft- und dedizierter Hardware oder aber als Kombination von dedizierter Firm- und Hardware (z.B. mit Hilfe eines FPGA) realisiert sein. Eine Realisierung durch reine Software wäre nur dann denkbar, wenn das zu überwachende medizinische Gerät die für die Realisierung nötige Hardware bereits enthält. Auf vielen Mikrocontrollern könnte z.B. eine vollständige Trennung vom unsicheren Bereich 11 b des Netzes dadurch erfolgen, dass die I/O-Pins, welche der Kommunikation mit dem sicheren Bereich 11a des Kommunikationsnetzes 11 zugeordnet sind, in einen passiven Modus geschaltet werden.

Im Folgenden werden einige mögliche Beispiele für Einsatzgebiete der erfindungsgemäßen Vorrichtung gegeben:

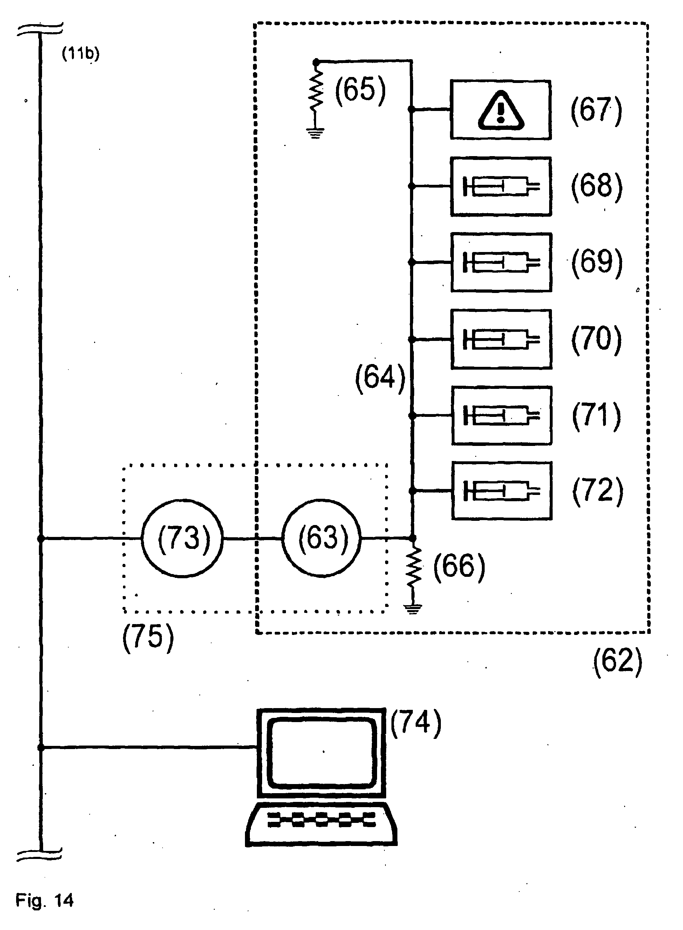

In Fig. 14 ist beispielhaft und schematisch die Absicherung eines Systems von Infusionspumpen durch das erfindungsgemäße Gerät dargestellt. Ein System von Infusionspumpen besteht in dargestellten Beispielen aus einem Kommunikationsmodul 63 welches an einen zentralen CAN-Bus 64 mit linearer Bustopologie und Abschlusswiderständen 65 und 66 angeschlossen ist, sowie aus einem zentralen Alarmierungssystem 67 und den Infusionspumpen 68 bis 72. Das Kommunikationsmodul 63 kann das auf dem CAN-Bus verwendete Kommunikationsprotokoll P₁ der Infusionspumpen 68 bis 72 in ein anderes, z. B. Ethernet-basiertes Kommunikationsprotokoll P₂ umsetzen. Dieses System von Infusionspumpen stellt im Sinne obiger Definitionen wegen der durch das System erbrachten zusätzlichen Leistungen (zentrale Kommunikation und zentrale Alarmierung) ein medizinisches Gerät 62 dar. Um es gegenüber möglichen Attacken aus dem unsicheren Bereich 11 b des Kommunikationsnetzes 11 abzusichern, wird eine erfindungsgemäße Vorrichtung 73 eingesetzt. Am unsicheren Bereich 11 b des Kommunikationsnetzes 11 ist ein PC 74 angeschlossen, der Tätigkeiten wie eine zentrale Überwachung der Alarmierung oder eine zentrale Verteilung von Therapiedaten vornehmen kann.

In diesem Fall ist es besonders vorteilhaft, eine erfindungsgemäße Vorrichtung mit bidirektionaler Paketfilterung und einer dem PC 74 bekannten Verschlüsselung zu wählen, um ein mögliches Bekannt werden von schutzwürdigen personenbezogenen Daten im unsicheren Teil des Netzes zu vermeiden und eine unbefugte Manipulation von Therapiedaten im unsicheren Bereich 11 b des Kommunikationsnetzes 11 verhindern zu können. Die Schalter 14a und 14 aus Fig. 3 können in diesem Fall ein Auftrennen der Ethernet-Leitung bei korrektem Abschluss durchführen. Auf diese Weise lässt sich eine physikalische Trennung der medizinischen Geräte 62 vom unsicheren Bereich 11 b des Netzwerks 11 vornehmen. Bei einer Realisierung entsprechend Fig. 3 bleibt die erfindungsgemäße Vorrichtung 73 mit dem unsicheren Bereich 11 b des Kommunikationsnetzes 11 verbunden und kann somit (ggf. nach einem eigenen Reset) erkennen, wann eine potentielle Attacke auf das medizinische Gerät 62 nicht mehr vorliegt. Erst in diesem Fall werden die Unterbrechungsschalter wieder geschlossen und das medizinische Gerät 62 wieder mit dem unsicheren Bereich 11 b des Kommunikationsnetzes 11 verbunden.

An diesem Beispiel ist erkennbar, dass Sicherheits- und/oder Kostenvorteile bei einer Realisierung entstehen können, wenn die erfindungsgemäße Vorrichtung 73 und das Kommunikationsmodul 63 einer Einheit 75 zusammengefasst werden, welche die Funktionen. beider Einzelgeräte vereinigt. Dieses kann z.B. bei vergleichbaren Kosten redundant ausgelegt werden, um ein höheres Maß an Sicherheit zu erzielen. Im Gegensatz zu der Realisierung mit zwei einzelnen Geräten lässt sich dadurch sogar der Fall, dass das Kommunikationsmodul 63 durch eine Fehlfunktion eine Attacke auf die anderen Subgeräte des medizinischen Geräts 62 ausführt, vermeiden. Eine derartige Vorrichtung 75 kann dann als eigenständiges Gerät oder als in das medizinische Gerät 62 integriert - z.B. als Einsteckkarte - realisiert werden. In beiden Fällen empfiehlt es sich, die Schalter 14a und 14b nun eine Trennung vom - bereits korrekt abgeschlossenen CAN-Bus 64 vornehmen zu lassen.

Als weiteres Beispiel sei der Anschluss einer Gruppe von Patientenmonitoren an einen Zentralmonitor genannt.

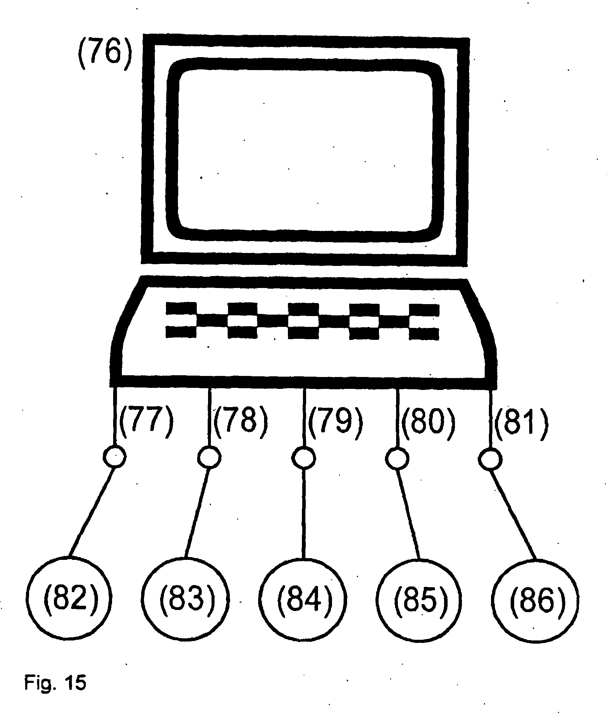

Besondere Vorteile können sich nämlich durch die Verwendung der erfindungsgemäßen Vorrichtung dann ergeben, wenn, ein bestehender älterer medizinischer Gerätepark - z.B. nach einem Umzug in ein anderes Gebäude - wieder vernetzt werden soll. Die älteren Geräte verfügen oft noch über serielle Schnittstellen (z.B. RS-485), die eine direkte Verbindung zwischen allen beteiligten Kommunikationspartnern erfordern. In Fig. 15 ist beispielhaft eine solche Vernetzung mehrerer Patientenmonitore, die mit einem Zentralmonitor 76 verbunden sind, dargestellt.

Der Zentralmonitor 76 ist über eine Reihe serieller RS-485-Schnittstellen 77 bis 81 mit den angeschlossenen Patientenmonitoren 82 bis 86 verbunden. Für jede einzelne Verbindung ist ein dediziertes Kabel zwischen dem Zentralenmonitor 76 und dem jeweiligen Patientenmonitor zu installieren.

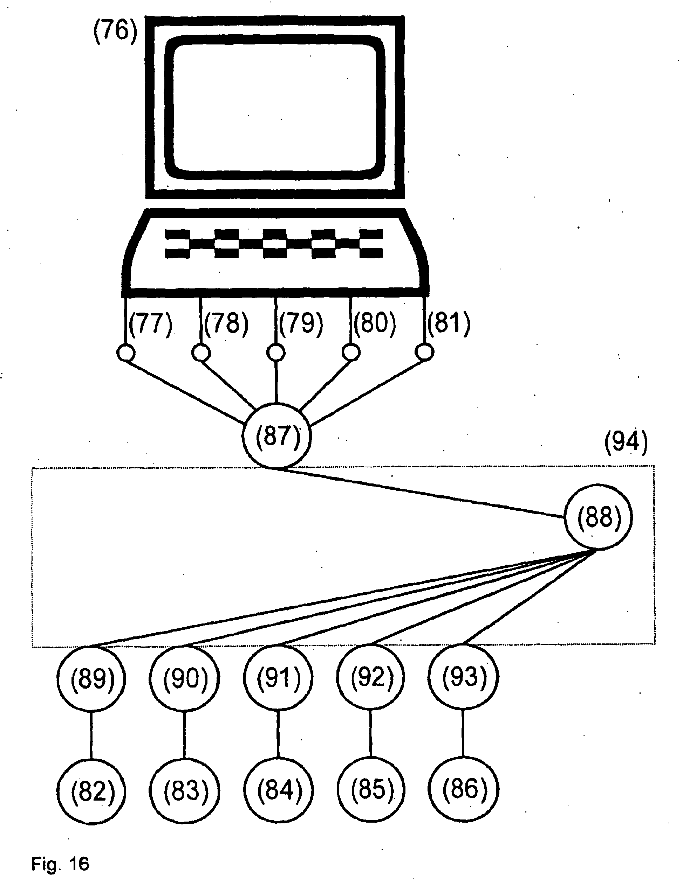

Stellt man sich vor dass innerhalb einer medizinischen Einrichtung auch noch viele andere Gruppen von medizinischen Geräten neben dem Patientenmonitoring vernetzt zur Verfügung gestellt werden sollen, sind die hohen Kosten solcher Vorgehensweisen offensichtlich. Demgegenüber ist in Fig. 16 die entstehende Struktur unter Verwendung der erfindungsgemäßen Vorrichtung und der Ausnutzung einer vorhandenen Ethernet-Infrastruktur dargestellt.

Der Zentralmonitor 76 ist nun an einer erfindungsgemäßen Vorrichtung 87, welches über mehrere RS-485-Schnittstellen verfügt, angeschlossen. Die erfindungsgemäße Vorrichtung 87 ist mit einem Ethernet-Switch 88 verbunden, an den die erfindungsgemäßen Vorrichtungen 89 bis 93 angeschlossen sind. Mit deren RS-485-Schnittstellen wiederum sind die Patientenmonitore 82 bis 86 verbunden.

Zunächst einmal sieht diese Form der Verbindung, hier der Verkabelung deutlich komplizierter aus, als die sternförmige Verkabelung aus Fig. 15. Es ist aber wichtig, sich zu vergegenwärtigen, dass die sich im gepunkteten Kasten 94 befindlichen Komponenten und Verbindungen des Kommunikationsnetzes 11 zur IT-Infrastruktur jeder modernen medizinischen Einrichtung gehören und damit ohne nennenswerte zusätzliche Kosten genutzt werden können. Außerdem ermöglicht eine derartige Vernetzungsstruktur ein sehr einfaches Bewegen von Patienten- und/oder Zentralmonitoren, da diese einfach zusammen mit ihren erfindungsgemäßen Vorrichtungen an anderen Ethernet-Dosen der medizinischen Einrichtung angesteckt werden können.

Der Einsatz der erfindungsgemäßen Vorrichtungen 87 und 89 bis 93 kann dabei z.B. folgendes sicherstellen:
- Die ursprünglichen RS-485-Kommunikationsdaten werden in umhüllenden TCP/IP-Paketen über das Ethernet transportiert,
- Die Daten sind gegenüber einer unbefugten Einsichtnahme im Ethernet durch eine Verschlüsselung geschützt,
- Die Daten sind gegenüber einer unbefugten Verfälschung im Ethernet geschützt,
- Durch sich verändernde Daten innerhalb der umhüllenden TCP/IP-Pakete können von anderen Netzwerkteilnehmern gespeicherte Pakete nicht zu einem späteren Zeitpunkt verwendet werden,
- Ein Zeitverhalten, das die Systeme 76 und 82 bis 86 bei einer seriellen Kommunikation problemlos erreichen können (z.B. Antwortzeiten auf Lebenszeichen, die aufgrund technischer Gegebenheiten oft sehr viel schneller, als es aus Risikogründen nötig wäre beantwortet und damit auch erwartet werden), welches aber prinzipbedingt bei einem Transport über das Ethernet nicht garantiert werden kann, wird von den erfindungsgemäßen Vorrichtungen durch Caching und Proxying garantiert. Erst beim Erkennen einer tatsächlichen Störung der Kommunikation (durch das Ausbleiben der Lebenszeichen des anderen medizinischen Geräts) werden die Antworten auf die Lebenszeichen nicht mehr von den erfindungsgemäßen Vorrichtungen gegeben,
- Verschiedene Gruppen von Patienten- und Zentralmonitoren, wie sie z.B. von verschiedenen Stationen herrühren, können sich innerhalb des Kommunikationsnetzes 11 der medizinischen Einrichtung nicht gegenseitig beeinflussen,
- Bei Aufkommen von potentiell gefährlichem Datenverkehr im Ethernet werden die Patientenmonitore und der Zentralmonitor vom Ethernet abgetrennt. Dies macht den Zentralmonitor betriebsunfähig, erhält jedoch die lokale Funktionsfähigkeit der Patientenmonitore.

Anhand von Fig. 16 ist erkennbar, dass die erfindungsgemäße Vorrichtung sinnvollerweise in einer Vielzahl unterschiedlicher Konfigurationen verfügbar gemacht werden sollte. Solche Konfigurationen können sich z.B. unterscheiden in:
- Der Art der verwendeten physikalischen Übertragungsprinzipien auf Ein- oder Ausgangsseite,
- der Art des verwendeten Kommunikationsprotokolls,
- der Art des verwendeten Applikationsprotokolls,
- der Anzahl unterschiedlicher Ein- und Ausgänge der erfindungsgemäßen Vorrichtung,
- den zeitlichen und/oder logischen Nebenbedingungen zum Auftrennen der Unterbrechungsschalter,
- den zeitlichen und/oder logischen Nebenbedingungen zum Schließen der Unterbrechungsschalter und
- den zeitlichen und/oder logischen Nebenbedingungen zum Durchführen eine Reset des erfindungsgemäßen Geräts.

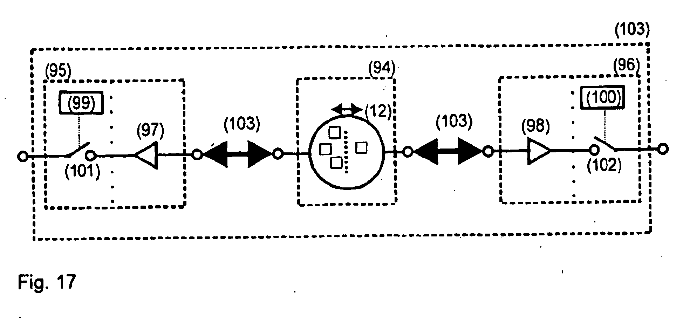

Eine solche Vielzahl möglicher Konfigurationen lässt sich sinnvoll herstellen, indem modulare Konzepte für die erfindungsgemäßen Vorrichtung eingesetzt werden. Ein Beispiel für eine derartige modulare erfindungsgemäße Vorrichtung ist in Fig. 17 dargestellt.

Die modulare erfindungsgemäße Vorrichtung 103 setzt sich zusammen aus dem Paketfilter-Modul 94 welches den bidirektionalen Paketfilter 12 enthält, sowie zwei Treiber-Modulen 95 und 96, welche jeweils selbständig auf einer Seite des Paketfiltermoduls über eine Kommunikationsschnittstelle angeschlossen sind. Die Treibermodule 95 und 96 enthalten je einen Treiber 97 bzw. 98, der eine Umwandlung in die benötigten physikalischen Medien und ggf. eine Implementierung der unteren Ebenen des Kommunikationsprotokolls vornimmt. Außerdem enthalten die Treibermodule 95 und 96 je eine Sicherungslogik 99 bzw. 100, welche die Unterbrechungsschalter 101 bzw. 102 ansteuern und ggf. einen Reset der erfindungsgemäßen Vorrichtung 103 auslösen können.

Ein so gestalteter modularer Aufbau der erfindungsgemäßen Vorrichtung vereinfacht die Anpassung an verschiedene Anwendungsfälle deutlich. Es ist in diesem Fall besonders sinnvoll, auch die Software der erfindungsgemäßen Vorrichtung modular aufzubauen, z.B. indem eine Treiber-Architektur zur Ansteuerung der verschiedenen Treibermodule implementiert wird. Ebenfalls sehr vorteilhaft ist die Entwicklung eines Codegenerators oder einer API/eines Frameworks, mit welchem die verschiedenen Aufgaben der Paketfilter auf unterschiedlichen Ebenen des ISO/OSI-Modells bzw. einer Applikations-Protokoll-Ebene einfach zu implementieren sind, unabhängig von dem genauen Aufbau der erfindungsgemäßen Vorrichtung.

In Abwandlungen der in Fig. 17 dargestellten modularen erfindungsgemäßen Vorrichtung kann es - insbesondere bei einer redundanten Realisierung - vorteilhaft sein, wenn die Sicherungslogiken 99 und 100 teilweise im Paketfiltermodul 94 enthalten sind, und über die jeweilige Kommunikationsschnittstelle die in den Treibermodulen enthaltenen vereinfachten Teile der Steuerungslogik zu einem Öffnen bzw. Schließen der jeweiligen Unterbrechungsschalter anregen können.

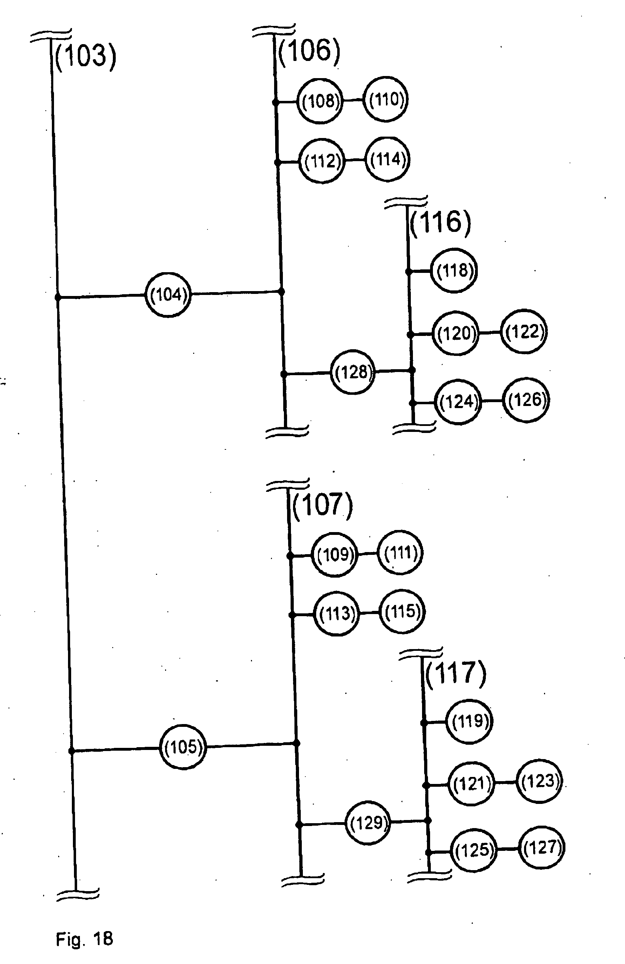

Ein Einsatz der erfindungsgemäßen Vorrichtung innerhalb einer medizinischen Einrichtung kann bei aus mehreren vernetzten Subnetzen bestehenden medizinischen Geräten/Systemen zusammen mit bekannten Netzwerktechnologien zu hierarchischen Absicherungen von Teilbereichen des Kommunikationsnetzes 11 entsprechend ihrer Einteilung in verschiedene Sicherheitsbereiche genutzt werden. In Fig. 18 ist beispielhaft eine solche hierarchische Absicherung durch eine erfindungsgemäße Vorrichtung dargestellt.

Die Kommunikationsnetze 106 und 107 zweier Standorte einer medizinischen Einrichtung sind über zwei Geräte 104 und 105 mit Firewall- und VPN-Funktionalität über das Internet miteinander verbunden. Am Kommunikationsnetz 106 ist ein Zentralmonitor 110 über eine erfindungsgemäße Vorrichtung 108 und ein Abrechnungs-PC 114 über ein Gerät 112 mit Firewall- und VPN-Funktionalität angeschlossen. Über die erfindungsgemäße Vorrichtung 128 ist beispielhaft das hier als medizinisches Gerät zu betrachtende Intensivzimmer A an dessen Kommunikationsnetz 116 angeschlossen. Dort sind ein Verordnungs-PC 118 sowie über die erfindungsgemäßen Vorrichtungen 120 und 124 ein Infusionspumpensystem 122 bzw. ein Patientenmonitor 126 angeschlossen. Der Patientenmonitor 126 liefert die Patientendaten an den Zentralmonitor 110 um sie zusammen mit den Patientendaten anderer Patientenmonitore am Zentralmonitor beobachtbar zu machen. Der Verordnungs-PC 118 dient dazu, ganze Listen von Infusionsdaten an das Infusionspumpensystem 122 zur Verteilung an die einzelnen Pumpen zu übertragen. Das ermöglicht es, komfortabel alle Pumpen des Infusionspumpensystems 122 für einen Start oder eine Anpassung der Infusionstherapie zu konfigurieren. Mit dem Verordnungs-PC 118 können ggf. auch Daten an andere, sich im selben Intensivzimmer befindliche Infusionspumpensysteme gesendet werden. Am Kommunikationsnetz 107 des zweiten Standortes liegt die gleiche Struktur des Kommunikationsnetzes 11 vor. Dort ist ein Zentralmonitor 111 über eine erfindungsgemäße Vorrichtung und ein Abrechnungs-PC 115 über ein Gerät 113 mit Firewall- und VPN-Funktionalität angeschlossen. Über die erfindungsgemäße Vorrichtung 129 ist das Intensivzimmer B an dessen Kommunikationsnetz 117 angeschlossen. Innerhalb dessen ist ein Verordnungs-PC sowie über die erfindungsgemäßen Vorrichtungen 121 und 125 ein Infusionspumpensystem 123 bzw. ein Patientenmonitor 127 angeschlossen.

Diese Netzwerkarchitektur kann z.B. zu folgenden Kommunikationswegen genutzt werden:
- Standort A und Standort B teilen sich ein gemeinsames Kommunikationsnetz 11, welche aus zwei Teilen 106 und 107 besteht. Damit im Internet keine Daten unbefugt eingesehen oder manipuliert werden können, wird ein VPN genutzt. Die Firewalls in 104 und 105 sichern die beiden Kommunikationsnetze 11 gegen Attacken vom Internet aus.
- Die Abrechnungs-PCs 114 und 115 sind über ein VPN miteinander verbunden. Dieses verhindert, dass die vertraulichen abzugleichenden Daten von unbefugten innerhalb der Teile 106 und 107 Kommunikationsnetzes 11 eingesehen oder manipuliert werden können. Da ein nicht unerheblicher Teil von Malware und den davon hervorgerufenen Attacken auf ein Netzwerk über Datenträger hinter die Firewalls von Unternehmen gelangen, ist hier eine Absicherung des für die medizinische Einrichtung erfolgskritischen Bereichs des Kommunikationsnetzes 11 durch die Firewalls in den Geräten 112 und 113 sinnvoll.
- Innerhalb des Intensivzimmers A kommunizieren das Infusionspumpensystem 122 mit dem Verordnungs-PC 118 über das Kommunikationsnetz 116. Da an diesem Teil des Kommunikationsnetzes 11 auch andere, für das Infusionspumpensystem 122 potentiell gefährliche Geräte angeschlossen sein können - sogar der Verordnungs-PC könnte auf dem Weg über Datenträger mit Malware befallen sein -wird es durch eine erfindungsgemäße Vorrichtung 120 abgesichert.
- Ein Patientenmonitor, der durch die erfindungsgemäße Vorrichtung 124 abgesichert ist, kann mit dem Zentralenmonitor, der durch die erfindungsgemäße Vorrichtung 108 abgesichert ist, eine Kommunikation durchführen.
- Um den Einfluss anderer Kommunikationsteilnehmer im Kommunikationsnetz 106 zu minimieren, werden durch die erfindungsgemäße Vorrichtung 128 nur solche Kommunikationspakete in das Intensivzimmer A passieren gelassen, die für die dortigen medizinischen Geräte tatsächlich relevant sind.

Es werden nun einige Reaktionen auf mögliche Attacken dargelegt. Dabei wird davon ausgegangen, dass die Attacken alle am System angeschlossenen Geräte adressieren und von der Steuerungslogik der jeweiligen Vorrichtungen als so schwerwiegend erkannt werden, dass ein blosses Unterdücken von Kommunikationspaketen durch den jeweiligen Paketfilter nicht ausreichend erscheint, sondern der jeweilige Unterbrechungsschalter geöffnet wird.

Im Falle einer Attacke innerhalb des Kommunikationsnetzes 116 betätigt die erfindungsgemäße Vorrichtung 120 den Unterbrechungsschalter, sodass das Infusionspumpensystem 122 seine Infusionen ungestört fortsetzen kann, eine Kommunikation mit dem Verordnungs-PC 118 allerdings nicht mehr möglich ist. Die erfindungsgemäße Vorrichtung 124 betätigt in dieser Situation ebenfalls den Unterbrechungsschalter, sodass der Patientenmonitor 126 ebenfalls die Monitoring-Funktionalität ungestört verrichtet, allerdings eine Kommunikation mit dem Zentralenmonitor 110 nicht mehr möglich ist. Darüber hinaus betätigt die erfindungsgemäße Vorrichtung 128 ebenfalls den Unterbrechungsschalter und lässt somit keine Einflüsse der Attacke auf den Rest des Kommunikationsnetzes 106 zu. Damit ist die Funktionalität anderer Intensivzimmer, bei denen die Attacke nicht innerhalb des eigenen Netzes auftritt, ungestört sichergestellt, und auch der Zentralmonitor 110 kann mit den Patientenmonitoren der anderen Intensivzimmer ungestört kommunizieren.

Im Falle einer Attacke, die vom Kommunikationsnetz 106 ausgeht, werden die Unterbrechungsschalter der erfindungsgemäßen Vorrichtungen 112 und 128 betätigt. Der Zentralmonitor hat somit keine Verbindung zu dem Patientenmonitor 126 und auch nicht zu Patientenmonitoren in anderen Intensivzimmern. Die Kommunikation innerhalb des Kommunikationsnetzes 116 bleibt jedoch ungestört, sodass die Verwendung des Verordnungs-PC 118 zusammen mit dem Infusionspumpensystem 122 weiterhin vollständig verfügbar bleibt.

Ein derartiger hierarchischer Einsatz der erfindungsgemäßen Vorrichtungen ermöglicht es, im Falle von Attacken nur solche Funktionalität nicht zu erbringen, für die das unvermeidbar ist und gleichzeitig, die Rückwirkungen auf andere Teilnehmer im Kommunikationsnetz zu minimieren, und damit deren Verfügbarkeit zu erhöhen.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

- 1: Wohnung des Patienten
- 2: medizinische Einrichtung
- 3: WAN-Netzwerk
- 4: datenerfassendes medizinisches Gerät
- 5: LAN-Netzwerk
- 6: LAN-Netzwerk der medizinischen Einrichtung
- 7: auswertendes medizinisches Gerät
- 8: unsicherer Bereich des Kommunikationsnetzwerks aus Sicht der medizinischen Einrichtung
- 9: erfindungsgemäße Vorrichtung
- 10: medizinisches Gerät
- 11: Kommunikationsnetz
- 11a: sicherer Bereich
- 11 b: unsicherer Bereich
- 12: Paketfilter
- 13: Steuerungslogik
- 14: Unterbrechungsschalter
- 15, 16: medizinische Teilgeräte
- 18: biderektionaler Paketfilter
- 19: Patientenmonitorzentrale
- 20,21,22: Patientenmonitore
- 26: gesendetes Kommunikationspaket
- 27: modifiziertes Kommunikationspaket
- 28: zusätzliche Daten
- 29: von medizinischen Gerät verwendbares Kommunikationspaket
- 30: verschlüsselter Datenbereich
- 31: verschlüsseltes modifiziertes Kommunikationspaket
- 32: erfindungsgemäße Vorrichtung mit Unterbrechungsschalter des unsicheren Bereichs des Kommunikationsnetzes
- 33: erfindungsgemäße Vorrichtung mit Unterbrechungsschaltern auf beiden Seiten
- 35: Arbeitskanal
- 36: Überwachungskanal
- 37: erfindungsgemäße Vorrichtung mit zwei voneinander unabhängigen Subsystemen
- 38, 39: unabhängige Subsysteme
- 40, 41: Unterbrechungsschalter
- 42, 43: Steuerungslogik
- 44, 45: Paketfilter
- 46: Vergleicher
- 47: Kommunikationsweg zwischen den Subsystemen
- 48: diversitäres erfindungsgemäßes Gerät
- 49, 50: Unterbrechungsschalter
- 51, 52: erster und zweiter Kanal
- 53, 54: erstes und zweites Betriebssystem
- 55, 56: erste und zweite Firewall
- 57, 58: erster und zweiter Virenscanner
- 59, 60: erste und zweite Protokollanalyse
- 61: Vergleicher
- 62: medizinische Geräte
- 63: Kommunikationsmodul
- 64: CAN-Bus
- 65, 66: Abschlußwiderstände
- 67: Alamierungssystem
- 68-72: Infusionspumpen
- 73: erfindungsgemäße Vorrichtung
- 74: PC
- 75: Einheit aus erfindungsgemäßer Vorrichtung 75 und Kommunikationsmodul 63
- 76: Zentralmonitor
- 77 - 81: RS-485-Schnittstellen
- 82-86: Patientenmonitore
- 87: erfindungsgemäße Vorrichtung
- 88: Internet-Switch
- 89-93: erfindungsgemäße Vorrichtungen
- 94: Einheit aus Komponenten und Verbindungen des Kommunikationsnetzes
- 95, 96: Treibermodule
- 97,98: Treiber
- 99, 100: Sicherungslogik
- 101, 102: Unterbrechungsschalter
- 103: erfindungsgemäßes Gerät
- 104, 105: Geräte mit Firewall - und VPN-Funktionalität
- 106, 107: Kommunikationsnetze zweier Standorte einer medizinischen Einrichtung
- 108, 109: erfindungsgemäße Vorrichtung
- 110, 111: Zentralmonitor
- 112, 113: Gerät mit Firewall-und VPN-Funktionalität
- 114, 115: Abrechnungs-PC's
- 116, 117: Kommunikationsnetze
- 118, 119: Verordnungs-PC's
- 120, 121: erfindungsgemäße Vorrichtungen
- 122, 123: Infusionspumpensyteme
- 124, 125: erfindungsgemäße Vorrichtungen
- 126, 127: Patientenmonitore
- 128, 129: erfindungsgemäße Geräte

## Patentansprüche

1. Vorrichtung (9; 34; 37) zur Wechselwirkung mit einem medizinischen Gerät (10), welches zur Einbindung in ein Kommunikationsnetzwerk (11), das zumindest einen unsicheren Bereich (11b) sowie geräteseitig einen sicheren Bereich (11a) aufweist, geeignet ist, wobei die Vorrichtung (9) Übertragungsmittel (12; 44, 45) zur Übertragung von Kommunikationspaketen zu und von dem medizinischen Gerät (10) über das Kommunikationsnetzwerk (11) aufweist, daß sie Überwachungsmittel (13; 42, 43) aufweist, um den Zustand der Verbindung des Geräts (10) mit dem Netzwerk (11) zu überwachten, sowie daß sie Unterbrechungsmittel (14; 40, 41) aufweist, um eine bestehende Verbindung zwischen dem sicheren Bereich (11a) und dem unsicheren Bereich (11b) des Netzwerks (11) zu unterbrechen, falls während der Überwachung ein Zustand der Netzwerkverbindung detektiert wird, der eine Gefährdung für einen vom Gerät (10) behandelten Patienten respektive für die korrekte Funktion des Geräts (10) darstellt,
und wobei die Vorrichtung mindestens zwei diversitäre Kanäle (38, 39) mit jeweils eigenen Übertragungsmitteln (12; 44, 45), Überwachungsmitteln (13; 42, 43) sowie Unterbrechungsmitteln (14; 40, 41) aufweist,
**dadurch gekennzeichnet, dass**
jeder Kanal sowohl sich als auch den anderen Kanal unabhängig überwacht bei Feststellung eines Zustands der Netzwerkverbindung, der eine Gefährdung für den Patienten respektive für die korrekte Funktion des Geräts darstellt, vom unsicheren Bereich (11b) des Kommunikationsnetzwerks (11) trennt.

2. Vorrichtung gemäß Anspruch 1,
**dadurch gekennzeichnet, dass**
sie einen Vergleicher (46; 61) aufweist, der geeignet ist, die Ergebnisse einer Paketfilterung in jedem Kanal (38, 39) miteinander zu vergleichen und die zu übertragenden Kommunikationspakete nur bei deren Gleichheit in jedem Kanal (38, 39) weiterzuleiten.

3. Vorrichtung gemäß Anspruch 1,
**dadurch gekennzeichnet, dass**
die Übertragungsmittel (12; 44, 45) einen Paketfilter aufweisen, der eine Paketfilterung bei den zwischen dem unsicheren Bereich (11b) und dem sicheren Bereich (11a) des Kommunikationsnetzwerks (11) übertragenen Kommunikationspaketen ausführt, wobei der Paketfilter geeignet ist, für das medizinische Gerät (10) potentiell gefährdende Kommunikationspakete nicht passieren zu lassen, daß die Unterbrechungsmittel zumindest einen Unterbrechungsschalter (14; 40, 41) aufweisen, sowie daß die Überwachungsmittel (12) zumindest eine Steuerungslogik (13; 42, 43) aufweisen, die bei Feststellung eines Zustands der Netzwerkverbindung, der eine Gefährdung für einen Patienten oder für die korrekte Funktion des Geräts darstellt, die Unterbrechung des/der Unterbrechungsschalter (14; 40, 41) auslöst, um den unsicheren Bereich (11b) vom direkt an das medizinische Gerät (10) angeschlossenen sicheren Bereich (11a) des Kommunikationsnetzwerks (11) abzutrennen.

4. Vorrichtung gemäß dem vorhergehenden Anspruch,
**dadurch gekennzeichnet, dass**
der/die Paketfilter (12; 44, 45) eine bidirektionale Paketfilterung bei den zwischen dem unsicheren Bereich (11b) und dem sicheren Bereich (11a) des Kommunikationsnetzwerks (11) übertragenen Kommunikationspaketen ausführt/ausführen.

5. Vorrichtung gemäß dem Anspruch 3 oder 4,
**dadurch gekennzeichnet, dass**
der Paketfilter (12; 44, 45) teilweise oder vollständig die Überwachungsmittel (13; 42, 43) und/oder die Unterbrechungsmittel (13, 14) ersetzt, indem er bei Feststellung eines Zustands der Netzwerkverbindung, der eine Gefährdung für den Patienten respektive für die korrekte Funktion des Geräts darstellt, gefährdende Kommunikationspakete oder alle Kommunikationspakete nicht passieren läßt.

6. Vorrichtung gemäß dem vorhergehenden Anspruch,
**dadurch gekennzeichnet, dass**
die Trennung zwischen sicherem Bereich (11a) und unsicherem Bereich (11b) des Kommunikationsnetzwerks (11) als logische Segmentierung ausgeführt ist.

7. Vorrichtung gemäß einem der Ansprüche 1 bis 5,
**dadurch gekennzeichnet, dass**
die Trennung zwischen sicherem Bereich (11a) und unsicherem Bereich (11b) des Kommunikationsnetzwerks (11) als physikalische Segmentierung ausgeführt ist.

8. Vorrichtung gemäß dem vorhergehenden Anspruch,
**dadurch gekennzeichnet, dass**
die besagte physikalische Segmentierung durch unterschiedliche physikalische Übertragungswege im sicherem Bereich (11a) bzw. im unsicherem Bereich (11b) des Kommunikationsnetzwerks (11) oder durch unterschiedliche Instanzen desselben Übertragungsweges realisiert ist.

9. Vorrichtung gemäß dem Anspruch 7 oder 8,
**dadurch gekennzeichnet, dass**
das Kommunikationsnetzwerk (11) kabelgebundene und/oder funkgebundene Übertragungswege aufweist.

10. Vorrichtung gemäß dem vorhergehenden Anspruch,
**dadurch gekennzeichnet, dass**
der Übertragungsweg im sicherem Bereich (11a) des Kommunikationsnetzwerks (11) aus Lichtwellenleitern besteht.

11. Vorrichtung gemäß einem der Ansprüche 3 bis 10,
**dadurch gekennzeichnet, dass**
der Pakekfilter (12; 44, 45) geeignet ist, eine Übersetzung und/oder Verschlüsselung der im unsicheren Bereich (11b) respektive im sicheren Bereich (11a) des Kommunikationsnetzwerks (11) verwendeten Kommunikationsprotokolle derart vorzunehmen, daß im jeweils anderen Bereich des Kommunikationsnetzwerks (11) gegenüber den aus dem Ursprungsprotokoll stammenden Kommunikationspaketen modifizierte Kommunikationspakete verwendet werden.

12. Vorrichtung gemäß einem der vorhergehenden Ansprüche 3 bis 11,
**dadurch gekennzeichnet, dass**
die besagte Steuerungslogik (13) Mittel zur Durchführung einer statischen - und/oder einer dynamischen Prüfung der im Kommunikationsnetzwerk (11) zu übertragenden Daten besitzt, deren Ergebnis zur Aufrechterhaltung oder Trennung einer bestehenden Verbindung zwischen dem sicheren Bereich (11a) und dem unsicheren Bereich (11b) des Netzwerks (11) durch Auslösen des/der Unterbrechungsschalter(s) (14; 40, 41) führt.

13. Vorrichtung gemäß einem der vorhergehenden Ansprüche,
**dadurch gekennzeichnet, dass**
sich der/die Unterbrechungsschalter (14; 40, 41) auf der an den sicheren Bereich (11a) des Kommunikationsnetzwerks (11) angeschlossenen Seite und/oder auf der an den unsicheren Bereich (11b) des Kommunikationsnetzwerks (11) angeschlossenen Seite der Vorrichtung (9) befindet/befinden, um nur das medizinische Gerät (10) und/oder sowohl das medizinische Gerät (10) als auch die Vorrichtung (9) selbst vom unsicheren Bereich (11b) abtrennen zu können.

14. Vorrichtung gemäß einem der vorhergehenden Ansprüche,
**dadurch gekennzeichnet, dass**
sie eine redundante Architektur aufweist, indem in den Überwachungsmitteln (13; 42, 43) ein Modell der Funktion der Übertragungsmittel (12; 44, 45) integriert ist, das deren korrekte Funktionsweise zu überprüfen erlaubt.

15. Vorrichtung gemäß dem Anspruch 2,
**dadurch gekennzeichnet, dass**
sie in jedem Kanal (38, 39) eine Sicherheitslogik aufweist, die geeignet ist, bei Fest- , stellung eines Zustands der Netzwerkverbindung, der eine Gefährdung für den Patienten respektive für die korrekte Funktion des Geräts darstellt, unabhängig von der Sicherheitslogik jedes anderen Kanals den unsicheren Bereich (11b) des Kommunikationsnetzwerks (11) von dessen sicheren Bereich (11a) zu trennen.

16. Medizinisches Gerät (10), welches zur Einbindung in ein Kommunikationsnetzwerk (11), das zumindest einen unsicheren Bereich (11b) sowie geräteseitig einen sicheren Bereich (11a) aufweist, geeignet ist,
**dadurch gekennzeichnet, dass**
es eine Vorrichtung (9; 34; 37) laut einem der vorhergehenden Ansprüche aufweist.

17. Medizinisches Gerät gemäß dem vorhergehenden Anspruch,
**dadurch gekennzeichnet, dass**
es aus einer Infusionspumpe (62) oder aus einem Patientenmonitor besteht.

18. Medizinisches System mit einer Vielzahl von medizinischen Geräten (10) oder Teilgeräten (15, 16), welche zur Einbindung in ein Kommunikationsnetzwerk (11), das zumindest einen unsicheren Bereich (11b) sowie geräteseitig einen sicheren Bereich (11a) aufweist, geeignet ist,
**dadurch gekennzeichnet, dass**
das System zumindest eine Vorrichtung (9; 34; 37) laut einem der vorhergehenden Ansprüche 1 bis 15 aufweist.

19. Medizinisches System gemäß dem vorhergehenden Anspruch,
**dadurch gekennzeichnet, dass**
jedes einzelne Gerät (10) oder Teilgerät (15, 16) eine eigene Vorrichtung (9) laut einem der vorhergehenden Ansprüche 1 bis 15 aufweist.

20. Verfahren zur Steuerung einer Vorrichtung (9; 34; 37) zur Wechselwirkung mit einem medizinischen Gerät (10), welches zur Einbindung in ein Kommunikationsnetzwerk (11), das zumindest einen unsicheren Bereich (11b) sowie geräteseitig einen sicheren Bereich (11a) aufweist, geeignet ist, wobei das Verfahren die Übertragung von Kommunikationspaketen zu und von dem medizinischen Gerät (10) über das Kommunikationsnetzwerk (11) sicherstellt, daß es den Zustand der Verbindung des Geräts (10) zum Netzwerk (11) überwacht, und daß es eine bestehende Verbindung zwischen dem sicheren Bereich (11a) und dem unsicheren Bereich (11b) des Netzwerks (11) unterbricht, falls während der Überwachung ein Zustand der Netzwerkverbindung detektiert wird, der eine Gefährdung für den Patienten respektive für die korrekte Funktion des Geräts darstellt,
und wobei das Verfahren in mindestens zwei diversitären Kanälen (38, 39) mit jeweils eigener Übertragung, Überwachung sowie Unterbrechung arbeitet,
**dadurch gekennzeichnet, dass**
jeder Kanal sowohl sich als auch den anderen Kanal unabhängig überwacht bei Feststellung eines Zustands der Netzwerkverbindung, der eine Gefährdung für den Patienten respektive für die korrekte Funktion des Geräts darstellt, vom unsicheren Bereich (11b) des Kommunikationsnetzwerks (11) trennt.

21. Verfahren gemäß dem vorhergehenden Anspruch,
**dadurch gekennzeichnet,dass**
es bei der Übertragung der zwischen dem unsicheren Bereich (11b) und dem sicheren Bereich (11a) des Kommunikationsnetzwerks (11) übertragenen Kommunikationspakete eine Paketfilterung ausführt, wobei diese Filterung geeignet ist, für das medizinische Gerät (10) potentiell gefährdende Kommunikationspakete nicht passieren zu lassen.

22. Verfahren gemäß einem der Ansprüche 20 bis 21,
**dadurch gekennzeichnet,dass**
es eine bidirektionale Paketfilterung bei den zwischen dem unsicheren Bereich (11b) und dem sicheren Bereich (11a) des Kommunikationsnetzwerks (11) übertragenen Kommunikationspaketen ausführt.

23. Verfahren gemäß einem der Ansprüche 20 bis 22,
**dadurch gekennzeichnet,dass**
es eine logische Segmentierung zwischen sicherem Bereich (11a) und unsicherem Bereich (11b) des Kommunikationsnetzwerks (11) erlaubt.

24. Verfahren gemäß einem der Ansprüche 21 bis 23,
**dadurch gekennzeichnet, dass**
es durch die Paketfilterung im Rahmen des Übertragungsschritts teilweise oder vollständig die besagten Schritte der Überwachung und/oder der Unterbrechung zu ersetzen erlaubt, indem es während der Paketfilterung bei Feststellung eines Zustands der Netzwerkverbindung, der eine Gefährdung für den Patienten respektive für die korrekte Funktion des Geräts darstellt, gefährdende Kommunikationspakete oder alle Kommunikationspakete nicht passieren läßt.

25. Verfahren gemäß einem der Ansprüche 20 bis 24,
**dadurch gekennzeichnet, dass**
die Paketfilterung geeignet ist, eine Übersetzung und/oder Verschlüsselung der im unsicheren Bereich (11b) respektive im sicheren Bereich (11a) des Kommunikationsnetzwerks (11) verwendeten Kommunikationsprotokolle derart vorzunehmen, daß im jeweils anderen Bereich des Kommunikationsnetzwerks (11) gegenüber den aus dem Ursprungsprotokoll stammenden Kommunikationspaketen modifizierte Kommunikationspakete verwendet werden.

26. Verfahren gemäß einem der Ansprüche 20 bis 25,
**dadurch gekennzeichnet, dass**
während des Überwachungsschritts eine statische - und/oder eine dynamische Prüfung der im Kommunikationsnetzwerks (11) zu übertragenden Daten durchgeführt wird, deren Ergebnis zur Aufrechterhaltung oder Trennung einer bestehenden Verbindung zwischen dem sicheren Bereich (11a) und dem unsicheren Bereich (11b) des Netzwerks (11) führt.

27. Verfahren gemäß einem der Ansprüche 20 bis 26,
**dadurch gekennzeichnet, dass**
es redundant arbeitet, indem im Überwachungsschritt ein zumindest teilweises Modell der Funktion des Übertragungsschritt integriert ist, das deren korrekte Funktionsweise zu überprüfen erlaubt.

## Claims

1. Apparatus (9; 34; 37) for interacting with a medical device (10) which is suitable for connection into a communication network (11) which comprises at least one insecure area (11b) and a secure area (11a) on the device side, wherein the apparatus (9) comprises transmission means (12; 44, 45) for transmitting communication packets to and from the medical device (10) via the communication network (11), in that it comprises monitoring means (13; 42, 43) for monitoring the state of the connection of the device (10) to the network (11), and in that it comprises interruption means (14; 40, 41) for breaking an existing connection between the secure area (11a) and the insecure area (11b) of the network (11) if, during the monitoring process, a state of the network connection is detected which poses a risk to a patient treated with the device (10) or to the correct functioning of the device (10), wherein the apparatus comprises at least two diversitary channels (38, 39), each with their own transmission means (12; 44, 45), monitoring means (13; 42, 43) and interruption means (14; 40, 41) **characterised in that** each channel can monitor both itself and also the other channel independently and can separate from the insecure area (11b) of the communication network (11) when a state of the network connection which poses a risk to the patient or to the correct functioning of the device is detected.

2. Apparatus according to claim 1, **characterised in that** it comprises a comparator (46; 61) which is suitable for comparing the results of a packet filtering in each channel (38, 39) with one another and forwarding the communication packets to be transmitted only if they are the same in each channel (38, 39).

3. Apparatus according to claim 1, **characterised in that** the transmission means (12; 44, 45) comprise a packet filter which carries out packet filtering on the communication packets transmitted between the insecure area (11b) and the secure area (11a) of the communication network (11), wherein the packet filter is suitable for blocking communication packets which pose a potential risk to the medical device (10), **in that** the interruption means comprise at least one interruption switch (14; 40, 41), and **in that** the monitoring means (12) comprise at least one control logic (13; 42, 43) which, when a state of the network connection which poses a risk to a patient or to the correct functioning of the device is detected, triggers opening of the interruption switch(es) (14; 40, 41) so as to separate the insecure area (11b) from the secure area (11a) of the communication network (11) which is connected directly to the medical device (10).

4. Apparatus according to the preceding claim, **characterised in that** the packet filter(s) (12; 44, 45) carries (carry) out a bidirectional packet filtering on the communication packets transmitted between the insecure area (11b) and the secure area (11a) of the communication network (11).

5. Apparatus according to claim 3 or 4, **characterised in that** the packet filter (12; 44, 45) partially or completely replaces the monitoring means (13; 42, 43) and/or the interruption means (13, 14) by blocking harmful communication packets or all communication packets when a state of the network connection which poses a risk to the patient or to the correct functioning of the device is detected.

6. Apparatus according to the preceding claim, **characterised in that** the separation between the secure area (11a) and the insecure area (11b) of the communication network (11) is implemented as logical segmentation.

7. Apparatus according to one of claims 1 to 5, **characterised in that** the separation between the secure area (11a) and the insecure area (11b) of the communication network (11) is implemented as physical segmentation.

8. Apparatus according to the preceding claim, **characterised in that** said physical segmentation is achieved by different physical transmission paths in the secure area (11a) and in the insecure area (11b) of the communication network (11) or by different instances of the same transmission path.

9. Apparatus according to claim 7 or 8, **characterised in that** the communication network (11) comprises cable-based and/or radio-based transmission paths.

10. Apparatus according to the preceding claim, **characterised in that** the transmission path in the secure area (11a) of the communication network (11) consists of optical fibres.

11. Apparatus according to one of claims 3 to 10, **characterised in that** the packet filter (12; 44, 45) is suitable for carrying out a translation and/or encryption of the communication protocols used in the insecure area (11b) or in the secure area (11a) of the communication network (11) in such a way that communication packets that have been modified with respect to the communication packets originating from the original protocol are used in the respective other area of the communication network (11).

12. Apparatus according to one of the preceding claims 3 to 11, **characterised in that** said control logic (13) has means for carrying out a static and/or dynamic check of the data to be transmitted in the communication network (11), the result of which check leads to an existing connection between the secure area (11a) and the insecure area (11b) of the network (11) being maintained or cut by triggering the interruption switch(es) (14; 40, 41).

13. Apparatus according to one of the preceding claims, **characterised in that** the interruption switch(es) (14; 40, 41) is/are located on the side connected to the secure area (11a) of the communication network (11) and/or on the side of the apparatus (9) connected to the insecure area (11b) of the communication network (11), so as to be able to separate only the medical device (10) and/or both the medical device (10) and the apparatus (9) itself from the insecure area (11b).

14. Apparatus according to one of the preceding claims, **characterised in that** it comprises a redundant architecture by integrating in the monitoring means (13; 42, 43) a model of the function of the transmission means (12; 44; 45) which makes it possible to check the correct functioning thereof.

15. Apparatus according to claim 2, **characterised in that** it comprises in each channel (38, 39) a security logic which is suitable for separating the insecure area (11b) of the communication network (11) from the secure area (11a) thereof, independently of the security logic of any other channel, when a state of the network connection which poses a risk to the patient or to the correct functioning of the device is detected.

16. Medical device (10) which is suitable for connection into a communication network (11) which comprises at least one insecure area (11b) and a secure area (11a) on the device side, **characterised in that** it comprises an apparatus (9; 34; 37) according to one of the preceding claims.

17. Medical device according to the preceding claim, **characterised in that** it consists of an infusion pump (62) or of a patient monitor.

18. Medical system comprising a plurality of medical devices (10) or sub-devices (15, 16), which is suitable for connection into a communication network (11) which comprises at least one insecure area (11b) and a secure area (11a) on the device side, **characterised in that** the system comprises at least one apparatus (9; 34; 37) according to one of the preceding claims 1 to 15.

19. Medical system according to the preceding claim, **characterised in that** each individual device (10) or sub-device (15, 16) has its own apparatus (9) according to one of the preceding claims 1 to 15.

20. Method for controlling an apparatus (9; 34; 37) for interacting with a medical device (10) which is suitable for connection into a communication network (11) which comprises at least one insecure area (11b) and a secure area (11a) on the device side, wherein the method ensures the transmission of communication packets to and from the medical device (10) via the communication network (11), in that it monitors the state of the connection of the device (10) to the network (11), and in that it breaks an existing connection between the secure area (11a) and the insecure area (11b) of the network (11) if, during the monitoring process, a state of the network connection is detected which poses a risk to the patient or to the correct functioning of the device and wherein the method operates in at least two diversitary channels (38, 39), each with their own transmission, monitoring and breaking, **characterised in that** each channel can monitor both itself and also the other channel independently and can separate from the insecure area (11b) of the communication network (11) when a state of the network connection which poses a risk to the patient or to the correct functioning of the device is detected.

21. Method according to the preceding claim, **characterised in that** it carries out packet filtering during the transmission of the communication packets transmitted between the insecure area (11b) and the secure area (11a) of the communication network (11), wherein this filtering is suitable for blocking communication packets which pose a potential risk to the medical device (10).

22. Method according to one of claims 20 to 21, **characterised in that** it carries out a bidirectional packet filtering on the communication packets transmitted between the insecure area (11b) and the secure area (11a) of the communication network (11).

23. Method according to one of claims 20 to 22, **characterised in that** it allows logical segmentation between the secure area (11a) and the insecure area (11b) of the communication network (11).

24. Method according to one of claims 21 to 23, **characterised in that** it allows said monitoring and/or breaking steps to be partially or completely replaced by the packet filtering during the transmission step by blocking, during the packet filtering, harmful communication packets or all communication packets when a state of the network connection which poses a risk to the patient or to the correct functioning of the device is detected.

25. Method according to one of claims 20 to 24, **characterised in that** the packet filtering is suitable for carrying out a translation and/or encryption of the communication protocols used in the insecure area (11b) or in the secure area (11a) of the communication network (11) in such a way that communication packets that have been modified with respect to the communication packets originating from the original protocol are used in the respective other area of the communication network (11).

26. Method according to one of claims 20 to 25, **characterised in that**, during the monitoring step, a static and/or dynamic check of the data to be transmitted in the communication network (11) is carried out, the result of which check leads to an existing connection between the secure area (11a) and the insecure area (11b) of the network (11) being maintained or cut.

27. Method according to one of claims 20 to 26, **characterised in that** it operates redundantly by integrating in the monitoring step an at least partial model of the function of the transmission step which makes it possible to check the correct functioning thereof.

## Revendications

1. Dispositif (9 ; 34 ; 37) destiné à une interaction avec un appareil médical (10), approprié pour être intégré à un réseau de communication (11), qui présente au moins une zone peu sûre (11b) ainsi que, côté appareil, une zone sûre (11a), dans lequel le dispositif (9) présente des moyens de transmission (12 ; 44, 45) pour transmettre des paquets de communications à l'appareil médical (10) et en sens inverse via le réseau de communication (11), des moyens de surveillance (13 ; 42, 43) pour surveiller la situation de la connexion de l'appareil (10) avec le réseau (11) et des moyens d'interruption (14; 40, 41) pour interrompre une connexion existante entre la zone sûre (11a) et la zone peu sûre (11b) du réseau (11), au cas où, au cours de la surveillance, il est détecté une situation de la connexion du réseau qui représente un danger pour un patient traité par l'appareil (10) ou un fonctionnement correct de l'appareil (10), et
dans lequel le dispositif présente au moins deux canaux discrets (38, 39) avec respectivement leurs moyens de transmission propres (12 ; 44, 45), leurs moyens de surveillance propres (13 ; 42, 43) ainsi que leurs moyens d'interruption propres (14; 40, 41),
**caractérisé en ce que** chaque canal surveille non seulement lui-même, mais encore l'autre canal indépendamment et, lors de l'établissement d'une situation de la connexion de réseau, qui représente un danger pour le patient ou un fonctionnement correct de l'appareil, se séparent de la zone peu sûre (11b) du réseau de communication (11).

2. Dispositif selon la revendication 1,
**caractérisé en ce qu'**il présente un comparateur (46 ; 61), qui est approprié pour comparer l'un à l'autre les résultats d'un filtrage de paquets dans chaque canal (38, 39) et pour transférer les paquets de communication à transmettre uniquement lorsqu'ils sont uniformes dans chaque canal (38, 39).

3. Dispositif selon la revendication 1,
**caractérisé en ce que** les moyens de transmission (12 ; 44, 45) présentent un filtre de paquets, qui effectue un filtrage des paquets dans le cas de paquets de communication transmis entre la zone peu sûre (11b) et la zone sûre (11a) du réseau de communication (11), dans lequel le filtre de paquets est approprié pour ne pas laisser passer de paquets de communication potentiellement dangereux pour l'appareil médical (10), les moyens d'interruption (12) présentent au moins un commutateur d'interruption (14 ; 42, 43) et les moyens de surveillance (12) présentent au moins une logique de commande (13 ; 42, 43) qui, lors de l'établissement d'une situation de la connexion du réseau, qui représente un danger pour un patient ou pour le fonctionnement correct de l'appareil, déclenche l'interruption du ou des commutateurs d'interruption( 14 ; 40, 41) pour séparer la zone peu sûre (11b) de la zone sûre (11a) du réseau de communication (11) connectée directement à l'appareil médical (10).

4. Dispositif selon la revendication précédente,
**caractérisé en ce que** le ou les filtres de paquets (12 ; 44, 45) effectuent un filtrage bidirectionnel des paquets lorsque les paquets de communication sont transmis entre la zone peu sûre (11b) et la zone sûre (11a) du réseau de communication (11).

5. Dispositif selon la revendication 3 ou 4,
**caractérisé en ce que** le filtre de paquets (12 ; 44, 45) remplace en partie ou en totalité les moyens de surveillance (13 ; 42, 43) et/ou les moyens d'interruption (13, 14) en ne laissant pas passer, lors de l'établissement d'une situation de la connexion de réseau qui représente un danger pour le patient ou le fonctionnement correct de l'appareil, de paquets de communication dangereux ou tous les paquets de communication.

6. Dispositif selon la revendication précédente,
**caractérisé en ce que** la séparation entre la zone sûre (11a) et la zone peu sûre (11b) du réseau de communication (11) se fait par segmentation logique.

7. Dispositif selon l'une quelconque des revendications 1 à 5,
**caractérisé en ce que** la séparation entre la zone sûre (11a) et la zone peu sûre (11b) du réseau de communication (11) se fait par segmentation physique.

8. Dispositif selon la revendication précédente,
**caractérisé en ce que** ladite segmentation physique est réalisée par différentes voies de transmission physiques dans la zone sûre (11a) respectivement dans la zone peu sûre (11b) du réseau de communication (11) ou par différentes sections de la même voie de transmission.

9. Dispositif selon la revendication 7 ou 8,
**caractérisé en ce que** le réseau de communication (11) présente des voies de transmission reliées par câble et/ou reliées par radio.

10. Dispositif selon la revendication précédente,
**caractérisé en ce que** la voie de transmission dans la zone sûre (11a) du réseau de communication (11) est constituée de guides d'ondes optiques.

11. Dispositif selon l'une quelconque des revendications 3 à 10,
**caractérisé en ce que** le filtre de paquets (12 ; 44, 45) est approprié pour effectuer une transmission et/ou un codage du protocole de communication utilisé dans la zone peu sûre (11b) ou dans la zone sûre (11a) du réseau de communication (11) de manière à utiliser, dans respectivement l'autre zone du réseau de communication (11) des paquets de communication modifiés par rapport aux paquets de communication provenus du protocole d'origine.

12. Dispositif selon l'une quelconque des revendications 3 à 11,
**caractérisé en ce que** ladite logique de commande (13) possède des moyens pour réaliser un test statique - et/ou un test dynamique - des données à transmettre dans le réseau de communication (11), test(s) dont le résultat mène au maintien ou à la séparation d'une connexion existante entre la zone sûre (11a) et la zone peu sûre (11b) du réseau (11) par déclenchement du ou des commutateurs d'interruption (14 ; 40, 41).

13. Dispositif selon l'une quelconque des revendications,
**caractérisé en ce que** le ou les commutateurs d'interruption (14 ; 40, 41) se trouve(nt) du côté du dispositif (9) connecté à la zone sûre (11a) du réseau de communication (11) et/ou du côté du dispositif (9) connecté à la zone peu sûre (11b) du réseau de communication (11) pour pouvoir séparer uniquement l'appareil médical (10) et/ou aussi bien l'appareil médical (10) que le dispositif (9) lui-même de la zone peu sûre (11b).

14. Dispositif selon l'une quelconque des revendications précédentes,
**caractérisé en ce qu'**il présente une architecture redondante en intégrant aux moyens de transmission (13 ; 44, 45) un modèle de la fonction des moyens de transmission (12 ; 44, 45) qui permet de contrôler son fonctionnement correct.

15. Dispositif selon la revendication 2,
**caractérisé en ce que**, dans chaque canal (38, 39), il présente une logique de sécurité qui est appropriée, lors de l'établissement d'une situation de la connexion de réseau qui représente un danger pour le patient ou le fonctionnement correct de l'appareil, indépendamment de la logique de sécurité de chaque autre canal, pour séparer la zone peu sûre (11b) du réseau de communication (11) de sa zone sûre (11a).

16. Appareil médical (10), qui est approprié pour être intégré dans un réseau de communication (11) qui présente au moins une zone peu sûre (11b) ainsi que côté appareil une zone sûre (11a),
**caractérisé en ce qu'**il présente un dispositif (9 ; 34 ; 37) selon l'une quelconque des revendications précédentes.

17. Appareil médical selon la revendication précédente,
**caractérisé en ce qu'**il est constitué d'une pompe à perfusion (62) ou d'un moniteur de patient.

18. Système médical comportant une pluralité d'appareils médicaux (10) ou d'appareils médicaux partiels (15, 16), qui sont appropriés pour être intégrés à un réseau de communication (11) qui présente au moins une zone peu sûre (11b) ainsi que côté appareil une zone sûre (11a),
**caractérisé en ce que** le système présente au moins un dispositif (9 ; 34 ; 37) selon l'une quelconque des revendications 1 à 15.

19. Système médical selon la revendication précédente,
**caractérisé en ce que** chaque appareil individuel (10) ou appareil partiel (15, 16) présente un dispositif propre (9) selon l'une quelconque des revendications 1 à 15.

20. Procédé pour commander un dispositif (9 ; 34 ; 37) pour une interaction avec un appareil médical (10), qui est approprié pour être intégré à un réseau de communication (11), qui présente au moins une zone peu sûre (11b) ainsi que, côté appareil, une zone sûre (11a), dans lequel le procédé assure la transmission de paquets de communication à l'appareil médical (10) et en sens inverse via le réseau de communication (11), surveille la situation de la connexion de l'appareil (10) avec le réseau (11) et interrompt une connexion existante entre la zone sûre (11a) et la zone peu sûre (11b) du réseau (11) si, au cours de la surveillance, on détecte une situation de la connexion du réseau qui représente un danger pour le patient ou le fonctionnement correct de l'appareil, et
dans lequel le procédé opère dans au moins deux canaux discrets (38, 39) avec respectivement une transmission propre, une surveillance propre et une interruption propre,
**caractérisé en ce que** chaque canal surveille non seulement lui-même, mais encore l'autre canal indépendamment et, lors de l'établissement d'une situation de la connexion de réseau, qui représente un danger pour le patient ou un fonctionnement correct de l'appareil, se séparent de la zone peu sûre (11b) du réseau de communication (11).

21. Procédé selon la revendication précédente,
**caractérisé en ce qu'**il exécute, lors de la transmission des paquets de communication transmis entre la zone peu sûre (11b) et la zone sûre (11a) du réseau de communication (11) un filtrage de paquets, ce filtrage étant approprié pour ne pas laisser passer de paquets de communication potentiellement dangereux pour l'appareil médical (10).

22. Procédé selon l'une quelconque des revendications 20 à 21,
**caractérisé en ce qu'**il exécute un filtrage de paquets bidirectionnel dans le cas de paquets de communication transmis entre la zone peu sûre (11b) et la zone sûre (11a) du réseau de communication (11).

23. Procédé selon l'une quelconque des revendications 20 à 22, **caractérisé en ce qu'**il permet une segmentation logique entre la zone sûre (11a) et la zone peu sûre (11b) du réseau de communication (11).

24. Procédé selon l'une quelconque des revendications 21 à 23,
**caractérisé en ce qu'**il permet de remplacer par le filtrage de paquets dans le cadre de l'étape de transmission en partie ou en totalité lesdites étapes de surveillance et/ou d'interruption d'une situation de la connexion du réseau en ne laissant pas passer, au cours du filtrage de paquets, lors de l'établissement d'une situation de la connexion du réseau, qui représente un danger pour le patient ou le fonctionnement correct de l'appareil, de paquets de communication dangereux ou tous les paquets de communication.

25. Procédé selon l'une quelconque des revendications 20 à 24,
**caractérisé en ce que** le filtrage de paquets est approprié pour effectuer une transmission et/ou un codage des protocoles de communication utilisés dans la zone peu sûre (11b) ou dans la zone sûre (11a) du réseau de communication (11) de manière à utiliser, dans respectivement l'autre zone du réseau de communication (11), des paquets de communication modifiés par rapport aux paquets de communication provenus du protocole d'origine.

26. Procédé selon l'une quelconque des revendications 20 à 25,
**caractérisé en ce que**, au cours de l'étape de surveillance, on effectue sur les données à transmettre dans le réseau de communication (11) un test statique - et/ou un test dynamique -, dont le résultat mène au maintien ou à la séparation d'une connexion existante entre la zone sûre (11a) et la zone peu sûre (11b) du réseau (11).

27. Procédé selon l'une quelconque des revendications 20 à 26,
**caractérisé en ce qu'**il opère de manière redondante en intégrant à l'étape de surveillance un modèle au moins partiel de la fonction de l'étape de transmission, qui permet de contrôler son fonctionnement correct.
